(19) Europäisches Patentamt
European Patent Office
Office européen des brevets

(11) Publication number: 0 201 193 B1

(12) # EUROPEAN PATENT SPECIFICATION

(45) Date of publication of the patent specification: 24.10.90

(51) Int. Cl.⁵: C07C 233/23, C07D 213/75, A01N 37/22, A01N 37/34, A01N 43/40, A01N 43/36

(21) Application number: 86302434.5

(22) Date of filing: 02.04.86

(54) **Carboxanilides.**

(30) Priority: 05.04.85 US 720212

(43) Date of publication of application: 17.12.86 Bulletin 86/46

(45) Publication of the grant of the patent: 24.10.90 Bulletin 90/43

(84) Designated Contracting States: AT BE CH DE FR GB IT LI LU NL SE

(56) References cited:
DE-A- 3 211 987

CHEMICAL ABSTRACTS, vol. 91, no. 5, 30th July 1979, page 588, abstract no. 38968p, Columbus, Ohio, US; M.M. MURZA et al.: "Synthesis and properties of anilides of perfluorocarboxylic acids", & IZV. VYSSH. UCHEBN. ZAVED., KHIM. KHIM. TEKHNOL. 1979, 22(3), 267-70 000

(73) Proprietor: AIR PRODUCTS AND CHEMICALS, INC., Route no. 222, Trexlertown Pennsylvania 18087(US)

(72) Inventor: Urenovitch, Joseph Victor, P.O. Box 136 R.D. 2, Tanaqua Pennsylvania 18252(US)

(74) Representative: Tapping, Kenneth George et al, Erl Wood Manor, Windlesham Surrey, GU20 6PH(GB)

EP 0 201 193 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention provides novel insecticidal and arachnicidal fluorinated cycloalkanecarboxanilides of the formula:

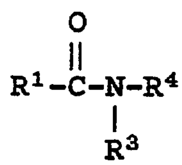

wherein $R^1$ represents

(1) perfluorocyclopropyl,
(2) perfluorocyclobutyl,
(3) perfluorocycloheptyl,
(4) perfluorodecahydronaphthyl,
(5) fluorocyclopentyl of the formula

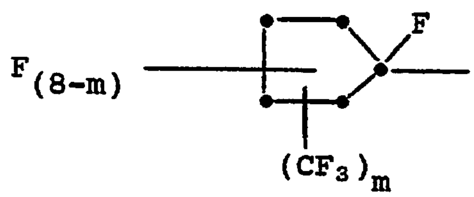

wherein m represents 0, 1, or 2, or
(6) fluorocyclohexyl of the formula

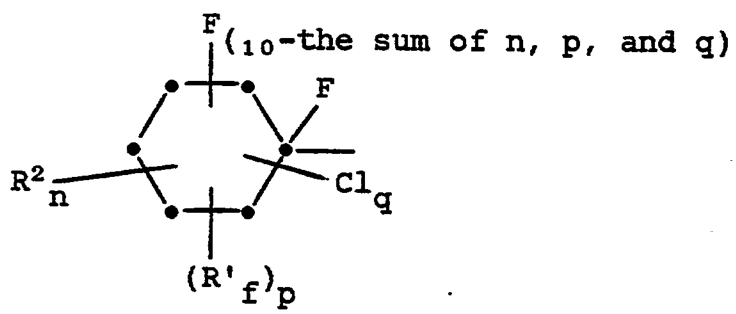

wherein
$R^2$ represents $-OR_f$ or

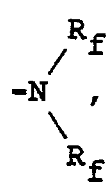

each $R_f$
independently represents perfluorinated loweralkyl of $C_1$-$C_3$, each $R'_f$ independently represents perfluorinated loweralkyl of $C_1$-$C_4$, n represents 0 or 1, each of p and q independently represents 0, 1, or 2, and the sum of n, p, and q is 0-2, inclusive;

$R^3$ represents hydrogen or methyl; and
$R^4$ represents phenyl substituted with

(1) a p-nitro group, or
(2) two to five independently selected $R^5$ groups, where $R^5$ is bromo, chloro, or fluoro, or
(3) two independently selected $R^6$ groups, where $R^6$ is iodo, nitro, cyano, trifluoromethyl, fluorosulfonyl, methylsulfonyl, ethylsulfonyl, carbomethoxy, or carboethoxy, or
(4) two groups, including one $R^5$ group and one $R^6$ group, or

(5) two groups, including one methyl group and one $R^5$, nitro, or fluorosulfonyl group,
(6) three groups, including two independently selected $R^5$ groups and one $R^6$ group,
(7) three groups, including two independently selected $R^6$ groups and one $R^5$ group,
(8) three groups, including two nitro groups and one trifluoromethyl group, or
(9) three groups, including two nitro groups at the 2- and 4-positions and a $C_1$-$C_4$ alkoxy or $C_1$-$C_4$ alkylthio group at the 3- or 5-position, or
(10) three groups, including one methyl group and two groups independently selected from $R^5$, nitro, and fluorosulfonyl, or
(11) two groups, including a thiocyanato group and a nitro, $R^5$, or iodo group, or

$R^4$ represents naphthyl substituted with two or three independently selected $R^5$ or $R^6$ groups, or $R^4$ represents 5-nitro-2-pyridyl;
and the sodium, potassium, and ammonium salts thereof, wherein ammonium is of the following formula

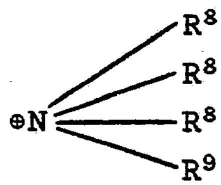

wherein each $R^8$ independently represents alkyl of $C_1$-$C_{20}$, benzyl, 2-hydroxyethyl, 2-hydroxypropyl, or 3-hydroxypropyl, and $R^9$ represents hydrogen or $R^8$, the total number of carbon atoms in all $R^8$ and $R^9$ groups being from 12 to 60, except that when one or more $R^8$ represents 2-hydroxyethyl, 2-hydroxypropyl, or 3-hydroxypropyl, the total number of carbon atoms in all $R^8$ and $R^9$ groups is from 6 to 60.

These compounds exhibit excellent insecticidal and arachnicidal activity. Accordingly, the present invention is also concerned with novel methods employing and novel formulations comprising the present compounds for the control of insects and arachnids.

Synthesis

All of the present fluorinated cycloalkanecarboxanilides are prepared in conventional procedures for the synthesis of carboxanilides. However, a preferred method of synthesis is the reaction of an acyl halide and an aniline, 1-aminonaphthalene, or 2-amino-5-nitropyridine, in accordance with the following scheme:

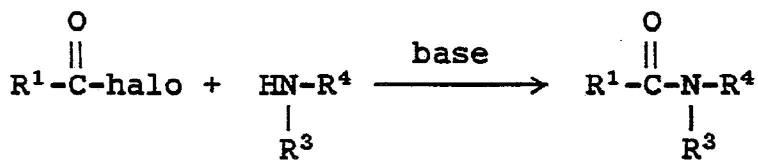


and although other conventional methods are believed to be available, no advantage over the foregoing method is expected.

The acyl halide is preferably an acyl fluoride. The acyl fluorides are obtained directly in the electrochemical fluorination process by which the fluorinated cycloalkyl ($R^1$) group is achieved, and thus require no additional reaction prior to their use in preparing the compounds of the present invention.

In carrying out the present reaction of acyl fluoride and aniline, 1-aminonaphthalene, or 2-amino-5-nitropyridine, the reactants are conveniently combined in a reaction solvent. Various solvents can be employed, including toluene, acetonitrile, diethyl ether, tetrahydrofuran, and halogenated solvents such as methylene chloride. In general, diethyl ether and halogenated solvents are preferred. A halogenated solvent can sometimes serve as the solvent in a "one-pot" reaction to make the aniline starting material which is thereafter converted to the final product of the invention. In other particulars, the reaction is conventional. An HF acceptor is provided to the reaction mixture; typically triethylamine is used. The reaction consumes the reactants and the HF acceptor in approximately equimolar amounts. The reaction goes forward over a range of temperatures, such as from 10 to 110°C.; however, the reaction is most conveniently carried out at temperatures of from about 20 to 70°C. Workup of the reaction mixture to isolate the product is carried out in conventional procedures.

Those compounds of the present invention in which $R^3$ represents methyl are preferably prepared from the corresponding $R^3$=hydrogen compounds. This methylation reaction is carried out in any of the known conventional methods for methylation. Typically, methyl iodide is employed as the reagent, and the reaction is carried out in a suitable solvent, such as acetone, with a base such as potassium carbonate.

Equimolar amounts of the reactants are consumed, but the methyl iodide is preferably used in excess. The reaction goes forward over a wide range of temperatures, but is most conveniently carried out at room temperatures of about 25 to 35°C. Workup is by conventional procedures.

The present invention also includes salts of the parent compounds. These salts are prepared in entirely conventional methods. The sodium and potassium salts are prepared by reacting the corresponding parent compounds with sodium or potassium hydroxide; the ammonium salts can be obtained by reacting the parent compound with a compound of the formula

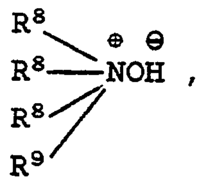

or by reacting a sodium salt of a present compound with

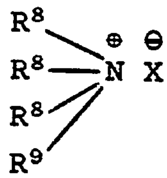

(where X = Br, Cl, or F).

The synthesis of the present compounds is further taught by the following illustrative examples.

EXAMPLE 1

2'-Bromo-4'-nitro-1,2,2,3,3,4,4,5,5,6,6-undecafluorocyclohexanecar oxanilide.

1,2,2,3,3,4,4,5,5,6,6-Undecafluorocyclohexanecarbonyl fluoride estimated to be of 60% purity (10.9 grams; 0.02 mole) and triethylamine (2 grams; 0.02 mole) were placed in 25 ml of diethyl ether. 2-Bromo-4-nitro-aniline (11.35 grams; 0.02 mole) in 75 ml of diethyl ether was added dropwise at ambient temperature of about 25°C. The reaction mixture was then stirred for 1½ hours. TLC showed no remaining aniline starting material. The reaction mixture was washed with water three times and with dilute sodium bicarbonate solution once, and thereafter dried. The solvent was then removed by evaporation. The product residue was chromatographed on silica gel HPLC with ethyl acetate:hexane 1:5, which yielded 14.5 grams of product containing occluded solvent. It was recrystallized from hexane and air dried, yielding 5.0 grams (48%) of purified product melting at 98-101°C. An additional 1 gram was obtained from the mother liquor for a total yield of 57%.

Analysis calculated for $C_{13}H_4BrF_{11}N_2O_3$
Theory: C, 29.74; H, 0.77; N, 5.34;
Found: C, 30.14; H, 0.91; N, 5.81.

Other compounds of the present invention, prepared in essentially the same procedures, are listed below.

EXAMPLE 2

4'-Nitro-1,2,2,3,3,4,4,5,5,6,6-undecafluorocyclohexanecarboxanilid , m.p., 166-169°C., yield 67%.

Analysis calculated for $C_{13}H_5F_{11}N_2O_3$
Theory: C, 35.00; H, 1.13; N, 6.28;
Found: C, 34.91; H, 1.10; N, 6.48.

EXAMPLE 3

2'-Iodo-4'-nitro-1,2,2,3,3,4,4,5,5,6,6-undecafluorocyclohexanecarboxanilide, m.p., 125-127°C., yield 38%.

Analysis calculated for $C_{13}H_4F_{11}IN_2O_3$
Theory: C, 27.35; H, 0.70; N, 4.89;
Found: C, 27.11; H, 0.67; N, 4.68.

EXAMPLE 4

2',4'-Dinitro-1,2,2,3,3,4,4,5,5,6,6-undecafluorocyclohexanecarboxanilide, m.p.,106-109°C., yield 14%.

Analysis calculated for $C_{13}H_4F_{11}N_3O_5$

Theory: C, 31.79; H, 0.82; N, 8.55, F, 42.54;

Found: C, 32.77; H, 0.84; N, 9.43, F, 43.66.

EXAMPLE 5

2'-Chloro-5'-nitro-1,2,2,3,3,4,4,5,5,6,6-undecafluorocyclohexanecarboxanilide, m.p., 115-118°C., yield 24%.

Analysis calculated for $C_{13}H_4ClF_{11}N_2O_3$

Theory: C, 32.53; H, 0.63; N, 5.84;

Found: C, 32.50; H, 0.73; N, 5.66;

Found: C, 32.22; H, 0.84; N, 5.62.

EXAMPLE 6

2'-Chloro-4'-nitro-1,2,2,3,3,4,4,5,5,6,6-undecafluorocyclohexanecarboxanilide, m.p., 90-93°C., yield 31%.

Analysis calculated for $C_{13}H_4ClF_{11}N_2O_3$

Theory: C, 32.48; H, 0.83; N, 5.82;

Found: C, 33.60; H, 1.01; N, 7.32.

EXAMPLE 7

2'-Methyl-4'-nitro-1,2,2,3,3,4,4,5,5,6,6-undecafluorocyclohexanecarboxanilide, m.p., 102-105°C., yield 38%.

Analysis calculated for $C_{14}H_7F_{11}N_2O_3$

Theory: C, 36.54; H, 1.53; N, 6.08;

Found: C, 43.69; H, 2.85; N, 10.44;

Found: C, 37.36; H, 1.72; N, 6.03

EXAMPLE 8

2'-Methyl-5'-nitro-1,2,2,3,3,4,4,5,5,6,6-undecafluorocyclohexanecarboxanilide, m.p., 107-109°C., yield 33%.

Analysis calculated for $C_{14}H_7F_{11}N_2O_3$

Theory: C, 36.54; H, 1.53; N, 6.09;

Found: C, 36.66; H, 1.28; N, 6.19.

EXAMPLE 9

2'-Cyano-4'-nitro-1,2,2,3,3,4,4,5,5,6,6-undecafluorocyclohexanecarboxanilide, m.p., 119-120°C., yield 20%.

Analysis calculated for $C_{14}H_4F_{11}N_3O_3$

Theory: C, 35.69; H, 0.86; N, 8.92;

Found: C, 35.94; H, 1.13; N, 8.66.

EXAMPLE 10

2'-(Trifluoromethyl)-4'-nitro-1,2,2,3,3,4,4,5,5,6,6-undecafluorocyclohexanecarboxanilide, m.p., 80-81°C., yield 34%.

EXAMPLE 11

2'-Fluoro-5'-nitro-1,2,2,3,3,4,4,5,5,6,6-undecafluorocyclohexanecarboxanilide, m.p., 101-102°C., yield 43%.

Analysis calculated for $C_{13}H_4F_{12}N_2O_3$

Theory: C, 33.71; H, 0.65; N, 6.05;

Found: C, 33.56; H, 0.66; N, 6.05.

EXAMPLE 12

2'-Cyano-4'-chloro-1,2,2,3,3,4,4,5,5,6,6-undecafluorocyclohexanecarboxanilide, m.p., 122-124°C.

EXAMPLE 13

2'-(Trifluoromethyl)-4'-bromo-1,2,2,3,3,4,4,5,5,6,6-undecafluorocyclohexanecarboxanilide, m.p., 104-106°C.
Analysis calculated for $C_{14}H_4BrF_{14}NO$
Theory: C, 30.70; H, 0.73; N, 2.56;
Found: C, 31.01; H, 0.69; N, 2.24.

EXAMPLE 14

2',3'-Dichloro-1,2,2,3,3,4,4,5,5,6,6-undecafluorocyclohexanecarboxanilide, m.p., 89-91°C.

EXAMPLE 15

3',4'-Dichloro-1,2,2,3,3,4,4,5,5,6,6-undecafluorocyclohexanecarboxanilide, m.p., 128-132°C.

EXAMPLE 16

3',4'-Dibromo-1,2,2,3,3,4,4,5,5,6,6-undecafluorocyclohexanecarboxanilide, m.p., 126-128°C., yield 39%.
Analysis calculated for $C_{13}H_4Br_2F_{11}NO$
Theory: C, 27.93; H, 0.72; N, 2.51;
Found: C, 28.15; H, 0.86; N, 2.77.

EXAMPLE 17

3'-Chloro-4'-fluoro-1,2,2,3,3,4,4,5,5,6,6-undecafluorocyclohexanecarboxanilide, m.p., 147-149°C., yield 9%.
Analysis calculated for $C_{13}H_4ClF_{12}NO$
Theory: C, 34.42; H, 0.89; N, 3.09;
Found: C, 34.30; H, 0.99; N, 3.15.

EXAMPLE 18

2',5'-Dibromo-1,2,2,3,3,4,4,5,5,6,6-undecafluorocyclohexanecarboxanilide, m.p., 110-113°C., yield 24%.
Analysis calculated for $C_{13}H_4Br_2F_{11}NO$
Theory: C, 27.93; H, 0.72; N, 2.50
Found: C, 28.09, H, 0.66; N, 2.52
Theory: Br, 28.59; F, 37.38;
Found: Br, 28.77; F, 37.54.

EXAMPLE 19

2',6'-Dichloro-1,2,2,3,3,4,4,5,5,6,6-undecafluorocyclohexanecarboxanilide, m.p., 159-162°C., yield 27%.
Analysis calculated for $C_{13}H_4Cl_2F_{11}NO$
Theory: C, 33.21; H, 0.85; N, 2.97;
Found: C, 33.04; H, 0.85; N, 2.90;
Theory: Cl, 15.08; F, 44.46;
Found: Cl, 14.99; F, 44.59.

EXAMPLE 20

2',5'-Dichloro-1,2,2,3,3,4,4,5,5,6,6-undecafluorocyclohexanecarboxanilide, m.p., 90-101°C., yield 26%.
Analysis calculated for $C_{13}H_4Cl_2F_{11}NO$
Theory: C, 33.21; H, 0.85; N, 2.97;
Found: C, 33.30; H, 0.97; N, 3.00
Theory: Cl, 15.08; F, 44.46;
Found: Cl, 15.12; F, 44.51.

EXAMPLE 21

2',4'-Dichloro-1,2,2,3,3,4,4,5,5,6,6-undecafluorocyclohexanecarboxanilide, m.p., 83-85°C., yield 8.5%.

Analysis calculated for $C_{13}H_4Cl_2F_{11}NO$
Theory: C, 33.21; H, 0.85; N, 2.97;
Found: C, 33.30; H, 0.85; N, 3.04;
Theory: Cl, 15.08; F, 44.46;
Found: Cl, 15.23; F, 44.24.

EXAMPLE 22

3',5'-Dichloro-1,2,2,3,3,4,4,5,5,6,6-undecafluorocyclohexanecarboxanilide, m.p., 125-128°C., yield 25%.

Analysis calculated for $C_{13}H_4Cl_2F_{11}NO$
Theory: C, 33.21; H, 0.85; N, 2.97;
Found: C, 33.25; H, 0.81; N, 3.02;
Theory: Cl, 15.08; F, 44.46
Found: Cl, 15.30; F, 44.67.

EXAMPLE 23

2',6'-Dibromo-1,2,2,3,3,4,4,5,5,6,6-undecafluorocyclohexanecarboxanilide, m.p., 143-145°C.

EXAMPLE 24

2',4'-Dibromo-1,2,2,3,3,4,4,5,5,6,6-undecafluorocyclohexanecarboxanilide, m.p., 84-86°C.

EXAMPLE 25

4',-Chloro-2'-nitro-1,2,2,3,3,4,4,5,5,6,6-undecafluorocyclohexanecarboxanilide, m.p., 66-69°C., yield 35%.

Analysis calculated for $C_{13}H_4ClF_{11}N_2O_3$
Theory: C, 32.49; H, 0.84; N, 5.83;
Found: C, 33.09; H, 0.97; N, 6.37.

EXAMPLE 26

3'-Nitro-4'-chloro-1,2,2,3,3,4,4,5,5,6,6-undecafluorocyclohexanecarboxanilide, m.p., 118-121°C., yield 67%.

Analysis calculated for $C_{13}H_4ClF_{11}N_2O_3$
Theory: C, 32.50; H, 0.83; N, 5.83;
Found: C, 32.71; H, 1.02; N, 6.10.

EXAMPLE 27

3'-Chloro-4'-nitro-1,2,2,3,3,4,4,5,5,6,6-undecafluorocyclohexanecarboxanilide, m.p., 141-144°C., yield 23%.

Analysis calculated for $C_{13}H_4ClF_{11}N_2O_3$
Theory: C, 32.49; H, 0.84; N, 5.83;
Found: C, 32.67; H, 1.05; N, 5.89.

EXAMPLE 28

3',5'-Dinitro-1,2,2,3,3,4,4,5,5,6,6-undecafluorocyclohexanecarboxanilide, m.p., 140-143°C., yield 22%.

Analysis calculated for $C_{13}H_4F_{11}N_3O_5$
Theory: C, 31.79; H, 0.82; N, 8.55;
Found: C, 31.61; H, 0.79; N, 9.01.

EXAMPLE 29

2',5'-Dinitro-1,2,2,3,3,4,4,5,5,6,6-undecafluorocyclohexanecarboxanilide, m.p., 125-128°C.

EXAMPLE 30

3',4'-Dinitro-1,2,2,3,3,4,4,5,5,6,6-undecafluorocyclohexanecarboxanilide, m.p., 118-121°C.

EXAMPLE 31

2',6'-Difluoro-1,2,2,3,3,4,4,5,5,6,6-undecafluorocyclohexanecarboxanilide, m.p., 88-90°C.

EXAMPLE 32

3'-(Trifluoromethyl)-4'-chloro-1,2,2,3,3,4,4,5,5,6,6-undecafluorocyclohexanecarboxanilide, m.p., 114-116°C.

EXAMPLE 33

2'-Chloro-5'-(trifluoromethyl)-1,2,2,3,3,4,4,5,5,6,6-undecafluorocyclohexanecarboxanilide, m.p., 81-84°C., yield 51%.
Analysis calculated for $C_{14}H_4ClF_{14}NO$
Theory: C, 33.50; H, 0.80; N, 2.79;
Found: C, 33.69; H, 0.84; N, 2.60.
Found: C, 33.51; H, 0.89; N, 2.59.

EXAMPLE 34

3',5'-Bis(trifluoromethyl)-1,2,2,3,3,4,4,5,5,6,6-undecafluorocyclohexanecarboxanilide, m.p., 137-139°C., yield 29%.
Analysis calculated for $C_{15}H_4F_{17}NO$
Theory: C, 33.55; H, 0.75; N, 2.61;
Found: C, 33.80; H, 0.51; N, 2.79.

EXAMPLE 35

2'-Nitro-4'-cyano-1,2,2,3,3,4,4,5,5,6,6-undecafluorocyclohexanecarboxanilide, m.p., 153-154°C.
Analysis calculated for $C_{14}H_4F_{11}N_3O_3$
Theory: C, 35.70; H, 0.85; N, 8.92;
Found: C, 36.20; H, 0.67; N, 8.59.

EXAMPLE 36

2'-Nitro-4'-(trifluoromethyl)-1,2,2,3,3,4,4,5,5,6,6-undecafluorocyclohexanecarboxanilide.
Analysis calculated for $C_{14}H_4F_{14}N_2O_3$
Theory: C, 32.80; H, 0.78; N, 5.45;
Found: C, 32.77; H, 0.70; N, 5.59.

EXAMPLE 37

2'-Methyl-4'-bromo-1,2,2,3,3,4,4,5,5,6,6-undecafluorocyclohexanecarboxanilide, m.p., 97-99°C, yield 17%.
Analysis calculated for $C_{14}H_7BrF_{11}NO$
Theory: C, 34.00; H, 1.42; N, 2.84;
Found: C, 34.07; H, 1.31; N, 2.60.

EXAMPLE 38

2'-Bromo-4'-methyl-1,2,2,3,3,4,4,5,5,6,6-undecafluorocyclohexanecarboxanilide, m.p., 79-80°C.

EXAMPLE 39

3'-Chloro-4'-methyl-1,2,2,3,3,4,4,5,5,6,6-undecafluorocyclohexanecarboxanilide, m.p., 108-110°C.

EXAMPLE 40

2'-Methyl-5'-chloro-1,2,2,3,3,4,4,5,5,6,6-undecafluorocyclohexanecarboxanilide, m.p., 115-118°C., yield 11%.
Analysis calculated for $C_{14}H_7ClF_{11}NO$

Theory: C, 37.45; H, 1.57; N, 3.12;
Found: C, 37.57; H, 1.52; N, 2.91.

EXAMPLE 41

2'-Methyl-4'-chloro-1,2,2,3,3,4,4,5,5,6,6-undecafluorocyclohexanecarboxanilide, m.p., 75-78°C., yield 31%.
Analysis calculated for $C_{14}H_7ClF_{11}NO$
Theory: C, 37.45; H, 1.57; N, 3.12;
Found: C, 37.50; H, 1.47; N, 2.89.

EXAMPLE 42

2'-Methyl-3'-chloro-1,2,2,3,3,4,4,5,5,6,6-undecafluorocyclohexanecarboxanilide, m.p., 115-116°C., yield 32%.
Analysis calculated for $C_{14}H_7ClF_{11}NO$
Theory: C, 37.40; H, 1.57; N, 3.11;
Found: C, 37.51; H, 1.47; N, 3.10.

EXAMPLE 43

2'-Chloro-5'-methyl-1,2,2,3,3,4,4,5,5,6,6-undecafluorocyclohexanecarboxanilide, m.p., 55-58°C., yield 77%.
Analysis calculated for $C_{14}H_7ClF_{11}NO$
Theory: C, 37.45; H, 1.57; N, 3.12;
Found: C, 37.82; H, 1.42; N, 3.29.

EXAMPLE 44

2'-Nitro-4'-methyl-1,2,2,3,3,4,4,5,5,6,6-undecafluorocyclohexanecarboxanilide, m.p., 96-98°C., yield 40%.
Analysis calculated for $C_{14}H_7F_{11}N_2O_3$
Theory: C, 36.54; H, 1.53; N, 6.08;
Found: C, 37.29; H, 1.86; N, 6.73.

EXAMPLE 45

3'-Fluoro-4'-methyl-1,2,2,3,3,4,4,5,5,6,6-undecafluorocyclohexanecarboxanilide, m.p., 118-120°C., yield 14%.
Analysis calculated for $C_{14}H_7F_{12}NO$
Theory: C, 38.83; H, 1.67; N, 3.24;
Found: C, 38.83; H, 1.71; N, 3.02.
Found: C, 39.25; H, 1.74; N, 3.09.

EXAMPLE 46

3'-Methyl-4'-fluoro-1,2,2,3,3,4,4,5,5,6,6-undecafluorocyclohexanecarboxanilide, m.p., 123-125°C., yield 34%.
Analysis calculated for $C_{14}H_7F_{12}NO$
Theory: C, 38.83; H, 1.63; N, 3.24;
Found: C, 38.84; H, 1.68; N, 3.04.

EXAMPLE 47

2'-Methyl-5'-fluoro-1,2,2,3,3,4,4,5,5,6,6-undecafluorocyclohexanecarboxanilide, m.p., 105-108°C., yield 46%.
Analysis calculated for $C_{14}H_7F_{12}NO$
Theory: C, 38.83; H, 1.63; N, 3.24;
Found: C, 38.93; H, 1.49; N, 3.07.

EXAMPLE 48

2'-Methyl-3'-fluoro-1,2,2,3,3,4,4,5,5,6,6-undecafluorocyclohexanecarboxanilide, m.p., 124-126°C., yield 50%.
Analysis calculated for $C_{14}H_7F_{12}NO$

Theory: C, 38.82; H, 1.63; N, 3.23;
Found: C, 38.77; H, 1.46; N, 3.20.

EXAMPLE 49

2',5'-Dichloro-4'-nitro-1,2,2,3,3,4,4,5,5,6,6-undecafluorocyclohexanecarboxanilide, m.p., 99-100°C., yield 7%.
Analysis calculated for $C_{13}H_3Cl_2F_{11}N_2O_3$
Theory: C, 30.32; H, 0.59; N, 5.44;
Found: C, 30.31; H, 0.85; N, 5.33.

EXAMPLE 50

2'-Methyl-4'-nitro-5'-chloro-1,2,2,3,3,4,4,5,5,6,6-undecafluorocyclohexanecarboxanilide, m.p., 154-156°C., yield 33%.
Analysis calculated for $C_{14}H_6ClF_{11}N_2O_3$
Theory: C, 33.99; H, 1.22; N, 5.66;
Found: C, 34.18; H, 1.41; N, 5.56.

EXAMPLE 51

2',5'-Dichloro-4'-bromo-1,2,2,3,3,4,4,5,5,6,6-undecafluorocyclohexanecarboxanilide, m.p., 128-129.5°C.
Analysis calculated for $C_{13}H_3BrCl_2F_{11}NO$
Theory: C, 28.42; H, 0.55; N, 2.55;
Found: C, 28.48; H, 0.76; N, 2.80.

EXAMPLE 52

2',4',5'-Trichloro-1,2,2,3,3,4,4,5,5,6,6-undecafluorocyclohexanecarboxanilide, m.p., 127-128.5°C.
Analysis calculated for $C_{13}H_3Cl_3F_{11}NO$
Theory: C, 31.00; H, 0.60; N, 2.80;
Found: C, 30.99; H, 0.61; N, 2.82.

EXAMPLE 53

2',4'-Dinitro-6'-chloro-1,2,2,3,3,4,4,5,5,6,6-undecafluorocyclohexanecarboxanilide, m.p., 109-111°C.

EXAMPLE 54

2'-Nitro-4',6'-dichloro-1,2,2,3,3,4,4,5,5,6,6-undecafluorocyclohexanecarboxanilide, m.p., 95-97°C.

EXAMPLE 55

2',4'-Dinitro-5'-fluoro-1,2,2,3,3,4,4,5,5,6,6-undecafluorocyclohexanecarboxanilide, m.p., 126-128°C.

EXAMPLE 56

2',6'-Dichloro-4'-nitro-1,2,2,3,3,4,4,5,5,6,6-undecafluorocyclohexanecarboxanilide, m.p., 155-157°C.

EXAMPLE 57

2',4',6'-Trichloro-1,2,2,3,3,4,4,5,5,6,6-undecafluorocyclohexanecarboxanilide, m.p., 170-172°C., yield 39%.
Analysis calculated for $C_{13}H_3Cl_3F_{11}NO$
Theory: C, 30.98; H, 0.60; N, 2.78;
Found: C, 30.97; H, 0.52; N, 2.55.

EXAMPLE 58

3',5'-Dichloro-4'-bromo-1,2,2,3,3,4,4,5,5,6,6-undecafluorocyclohexanecarboxanilide, m.p., 171-172.5°C.
Analysis calculated for $C_{13}H_3BrCl_2F_{11}NO$
Theory: C, 28.44; H, 0.55; N, 2.55;
Found: C, 28.65; H, 0.81; N, 2.67.

EXAMPLE 59

3',4',5'-Trichloro-1,2,2,3,3,4,4,5,5,6,6-undecafluorocyclohexanecarboxanilide, m.p., 152-154°C.

EXAMPLE 60

2',4',6'-Tribromo-1,2,2,3,3,4,4,5,5,6,6-undecafluorocyclohexanecarboxanilide, m.p., 115-117°C.

EXAMPLE 61

2'-Nitro-4',6'-dichloro-1,2,2,3,3,4,4,5,5,6,6-undecafluorocyclohexanecarboxanilide, m.p., 97-99°C.

EXAMPLE 62

2'-Bromo-4'-(trifluoromethyl)-1,2,2,3,3,4,4,5,5,6,6-undecafluorocyclohexanecarboxanilide.
Analysis calculated for $C_{13}H_3F_{11}N_4O_7$
Theory: C, 29.12; H, 0.56; N, 10.45; F, 38.98;
Found: C, 30.37; H, 0.63; N, 10.95; F, 33.79.

EXAMPLE 63

2',6'-Dinitro-4'-(trifluoromethyl)-1,2,2,3,3,4,4,5,5,6,6-undecafluorocyclohexanecarboxanilide, yield of crude, 61%, m.p. after recrystallization, 173-175°C.
Analysis calculated for $C_{14}H_3F_{14}N_3O_5$
Theory: C, 30.07; H, 0.54; N, 7.51;
Found: C, 30.59; H, 0.40; N, 7.43.

EXAMPLE 64

2',3',4',5'-Tetrachloro-1,2,2,3,3,4,4,5,5,6,6-undecafluorocyclohexanecarboxanilide, m.p., 130°C., yield 19%.
Analysis calculated for $C_{13}H_2Cl_4F_{11}NO$
Theory: C, 28.97; H, 0.37; N, 2.60;
Found: C, 29.21; H, 0.60; N, 2.77.

EXAMPLE 65

2',3',4',5'-Tetrafluoro-1,2,2,3,3,4,4,5,5,6,6-undecafluorocyclohexanecarboxanilide, m.p., 55-59°C., yield 18%.
Analysis calculated for $C_{13}H_2F_{15}NO$
Theory: C, 33.01; H, 0.42; N, 2.96;
Found: C, 33.72; H, 0.51; N, 2.90.

EXAMPLE 66

2',3',5',6'-Tetrafluoro-1,2,2,3,3,4,4,5,5,6,6-undecafluorocyclohexanecarboxanilide, m.p., 115-117°C., yield 38%.
Analysis calculated for $C_{13}H_2F_{15}NO$
Theory: C, 33.01; H, 0.42; N, 2.96;
Found: C, 32.79; H, 0.48; N, 2.69.

EXAMPLE 67

2',3',4',5',6'-Pentafluoro-1,2,2,3,3,4,4,5,5,6,6-undecafluorocyclohexanecarboxanilide, m.p., 135°C., yield 19%.
Analysis calculated for $C_{13}HF_{16}NO$
Theory: C, 31.80; H, 0.21; N, 2.85;
Found: C, 31.65; H. 0.11; N, 2.63.

EXAMPLE 68

2',3',4',5',6'-Pentachloro-1,2,2,3,3,4,4,5,5,6,6-undecafluorocyclohexanecarboxanilide, m.p., 181-184°C., yield 38%.

EXAMPLE 69

N-(5-Nitro-2-pyridyl)-1,2,2,3,3,4,4,5,5,6,6-undecafluorocyclohexanecarboxamide, m.p., 110-112°C., yield 24%.

Analysis calculated for $C_{12}H_4F_{11}N_3O_3$
Theory: C, 32.29; H, 0.89; N, 9.35;
Found: C, 32.16; H, 0.74; N, 9.22;
Found: C, 32.27; H, 0.84; N, 9.23.

EXAMPLE 70

2'-Bromo-4'-nitro-4-(trifluoromethyl)-1,2,2,3,3,4,5,5,6,6-undecafluorocyclohexanecarboxanilide, m.p., 102-104°C., yield 1.7%.

Analysis calculated for $C_{14}H_4BrF_{13}N_2O_3$
Theory: C, 29.24; H, 0.70; N, 4.87;
Found: C, 29.53; H, 0.44; N, 4.83.

EXAMPLE 71

Mixture of 2'-bromo-4'-nitro-4-(trifluoromethyl)-1,2,2,3,3,4,5,5,6,6-decafluorocyclohexanecarboxanilide and 2'-bromo-4'-nitro-x,x-bis(trifluoromethyl)-x,x,x,x,x,x,x-heptafluorocyclopentanecarboxanilide, m.p., 93-95°C., yield 10%.

Analysis calculated for $C_{14}H_4BrF_{13}N_2O_3$
Theory: C, 29.24; H, 0.70; N, 4.87;
Found: C, 29.11; H, 0.74; N, 4.96.

Example 72

N-Methyl-2'-bromo-4'-nitro-1,2,2,3,3,4,4,5,5,6,6-undecafluorocyclohexanecarboxanilide

2'-Bromo-4'-nitro-1,2,2,3,3,4,4,5,5,6,6-undecafluorocyclohexanecar oxanilide (5.0 grams; 0.01 mole) and methyl iodide (15 ml, 0.24 mole) were combined in 40 ml of acetone and the reaction mixture was stirred over a weekend, about 60 hours. About 200 ml of water was added to the reaction mixture and the reaction mixture was then extracted with diethyl ether, dried, evaporated, and chromatographed on silica gel with ethyl acetate:pentane 1:4. TLC showed one spot, with a lower $R_f$ than the starting cyclohexanecarboxanilide, indicating conversion to the desired N-methyl-2'-bromo-4'-nitro-1,2,2,3,3,4,4,5,5,6,6-undecafluorocyclohexan carboxanilide. The yield was 2.5 grams of an oil (49% yield).

Analysis calculated for $C_{14}H_6BrF_{11}N_2O_3$
Theory: C, 31.19; H, 1.12; N, 5.20;
Found: C, 31.46; H, 0.82; N, 5.21

Example 73

2'-Bromo-4'-nitro-1,2,2,3,3,4,4,5,5,6,6-undecafluorocyclohexanecarboxanilide, sodium salt

2'-Bromo-4'-nitro-1,2,2,3,3,4,4,5,5,6,6-undecafluorocyclohexanecarboxanilide (2.6 grams; 0.005 mole) and sodium hydroxide (0.2 gram; 0.005 mole) were combined in 50 ml of acetone at room termperature of about 25°C. All volatiles and water were removed by evaporation. The solid residue was dissolved in hot toluene/ethyl acetate; the product did not crystallize but formed lumps. These were separated and vacuum dried. The yield was 2.1 gram (77%), m.p., 200°C. with decomposition.

Analysis calculated for $C_{13}H_3BrF_{11}N_2O_3Na$
Theory: C, 28.54; H, 0.55; N, 5.12;
Found: C, 28.84; H, 1.10; N, 4.90.

EXAMPLE 74

2'-Bromo-4'-nitro-1,2,2,3,3,4,4,5,5,6,6-undecafluorocyclohexanecarboxanilide, tetraethylammonium salt.

2'-Bromo-4'-nitro-1,2,2,3,3,4,4,5,5,6,6-undecafluorocyclohexanecar oxanilide (2.6 grams; 0.005 mole) was dissolved in 25 ml. of acetone and 2N sodium hydroxide (0.2 gram; 0.005 mole) was added all at once. Tetraethylammonium bromide (1.1 gram; 0.005 mole) was then added and the reaction mixture was stirred until it became a single phase. The reaction mixture was poured into ice/water, extracted with methylene chloride/brine, dried over magnesium sulfate, and evaporated. NMR showed a mixture of the intended salt and the parent compound. The residue was therefore dissolved in acetone, retreated with sodium hydroxide and tetraethylammonium bromide, and worked up as before, yielding 1.5 grams (46% yield) of the 2'-bromo-4'-nitro-1,2,2,3,3,4,4,5,5,6,6-undecafluorocyclohexanecarboxanilide, tetraethylammonium salt as an oil.

EXAMPLE 75

2'-Bromo-4'-nitro-1,2,2,3,3,4,4,5,5,6,6-undecafluorocyclohexanecarboxanilide, tetra-n-propylammonium salt.

2'-Bromo-4'-nitro-1,2,2,3,3,4,4,5,5,6,6-undecafluorocyclohexanecarboxanilide (2.6 grams; 0.005 mole) was dissolved in 50 ml. of acetone and 1N sodium hydroxide (5.0 ml., 0.005 mole) was added all at once. Tetrapropylammonium bromide (1.35 gram; 0.005 mole) was then added and the reaction mixture was stirred until it became one phase. The reaction mixture was then poured over ice/water, and an oily solid separated. It was extracted with diethyl ether, dried over magnesium sulfate, evaporated, and crystallized at -10°C from water/ethanol. 0.9 gram (25% yield) of the tetra-n-propylammonium salt was obtained, m.p., 86-87°C.

Employing essentially the same procedures as the preceding two examples, the following additional salts were obtained. The identity of the salts was verified by H-NMR.

EXAMPLE 76

2'-Bromo-4'-nitro-1,2,2,3,3,4,4,5,5,6,6-undecafluorocyclohexanecarboxanilide, tri-n-butylmethylammonium salt, m.p., 129-130°C., yield 56%.

Analysis calculated for $C_{26}H_{33}BrF_{11}N_3O_3.H_2O$
Theory: C, 42.00; H, 4.71; N, 5.65;
Found: C, 43.02; H, 4.51; N, 6.12.

EXAMPLE 77

2'-Bromo-4'-nitro-1,2,2,3,3,4,4,5,5,6,6-undecafluorocyclohexanecarboxanilide, tetra-n-butylammonium salt, m.p., 94-97°C., yield 94%.

Analysis calculated for $C_{29}H_{39}BrF_{11}N_3O_3.H_2O$
Theory: C, 44.40; H, 5.27; N, 5.36;
Found: C, 44.66; H, 5.03; N, 5.46.

EXAMPLE 78

2'-Bromo-4'-nitro-1,2,2,3,3,4,4,5,5,6,6-undecafluorocyclohexanecarboxanilide, tetra-n-pentylammonium salt, m.p., 80-81°C., yield 85%.

Analysis calculated for $C_{33}H_{48}BrF_{11}N_3O_3.H_2O$
Theory: C, 47.21; H, 5.76; N, 5.00;
Found: C, 47.46; H, 5.61; N, 4.79.

EXAMPLE 79

2'-Bromo-4'-nitro-1,2,2,3,3,4,4,5,5,6,6-undecafluorocyclohexanecarboxanilide, tetra-n-hexylammonium salt, an oil, yield 53%.

Analysis calculated for $C_{37}H_{55}BrF_{11}N_3O_3.H_2O$
Theory: C, 49.56; H, 6.41; N, 4.69;
Found: C, 51.38; H, 6.73; N, 4.75.

EXAMPLE 80

2'-Bromo-4'-nitro-1,2,2,3,3,4,4,5,5,6,6-undecafluorocyclohexanecarboxanilide, tetra-n-heptylammonium salt, an oil, yield 51%.

Analysis calculated for $C_{41}H_{63}BrF_{11}N_3O_3.H_2O$
Theory: C, 51.68; H, 6.88; N, 4.41;
Found: C, 53.21; H, 7.17; N, 4.29.

EXAMPLE 81

2'-Bromo-4'-nitro-1,2,2,3,3,4,4,5,5,6,6-undecafluorocyclohexanecarboxanilide, benzyltriethylammonium salt, m.p., 77-82°C., yield 77%.

Analysis calculated for $C_{26}H_{25}BrF_{11}N_3O_3.H_2O$
Theory: C, 42.44; H, 3.67; N, 5.71;
Found: C, 41.79; H, 2.90; N, 5.85.

EXAMPLE 82

2'-Bromo-4'-nitro-1,2,2,3,3,4,4,5,5,6,6-undecafluorocyclohexanecarboxanilide, hexadecyltrimethylammonium salt, m.p., 53-55°C., yield 60%.

Analysis calculated for $C_{32}H_{45}BrF_{11}N_3O_3.H_2O$
Theory: C, 46.50; H, 5.73; N, 5.08;
Found: C, 47.57; H, 4.84; N, 4.97.

EXAMPLE 83

2'-Bromo-4'-nitro-1,2,2,3,3,4,4,5,5,6,6-undecafluorocyclohexanecarboxanilide, octadecyltrimethylammonium salt, m.p., 48-53°C., yield 38%.

Analysis calculated for $C_{34}H_{49}BrF_{11}N_3O_3.H_2O$
Theory: C, 47.78; H, 6.01; N, 4.92;
Found: C, 47.54; H, 5.83; N, 4.74.

EXAMPLE 84

2'-Bromo-4'-nitro-1,2,2,3,3,4,4,5,5,6,6-undecafluorocyclohexanecarboxanilide, benzyltri-n-butylammonium salt, an oil; yield 42%.

Analysis calculated for $C_{32}H_{37}BrF_{11}N_3O_3.H_2O$
Theory: C, 47.59; H, 4.78; N, 5.24;
Found: C, 49.97; H, 5.70; N, 4.90.

EXAMPLE 85

2'-Bromo-4'-nitro-1,2,2,3,3,4,4,5,5,6,6-undecafluorocyclohexanecarboxanilide, dimethylbis-($C_{14}$-$C_{18}$) ammonium salt, an oil, yield 39%

EXAMPLE 86

2'-Bromo-4'-nitro-1,2,2,3,3,4,4,5,5,6,6-undecafluorocyclohexanecarboxanilide, methyltri-n-octylammonium salt, an oil, yield 53%.

Analysis calculated for $C_{38}H_{57}BrF_{11}N_3O_3.H_2O$
Theory: C, 50.17; H, 6.43; N, 4.62;
Found: C, 50.47; H, 6.63; N, 4.34.

EXAMPLE 87

2'-Bromo-4'-nitro-1,2,2,3,3,4,4,5,5,6,6-undecafluorocyclohexanecarboxanilide, methyltris-($C_8$-$C_{10}$) ammonium salt, an oil, yield 45%.

EXAMPLE 88

2'-Bromo-4'-nitro-1,2,2,3,3,4,4,5,5,6,6-undecafluorocyclohexanecarboxanilide, dimethylbis-($C_{10}$-$C_{18}$) ammonium salt, an oil, yield 37%.

EXAMPLE 89

2'-Chloro-4'-nitro-1,2,2,3,3,4,4,5,5,6,6-undecafluorocyclohexanecarboxanilide, tetra-n-propylammonium salt, m.p., 65-69°C., yield 52%.

Analysis calculated for $C_{25}H_{31}ClF_{11}N_3O_3.H_2O$
Theory: C, 43.90; H, 4.86; N, 6.14;
Found: C, 43.88; H, 4.61; N, 5.99.

EXAMPLE 90

2'-Chloro-4'-nitro-1,2,2,3,3,4,4,5,5,6,6-undecafluorocyclohexanecarboxanilide, tetra-n-butylammonium salt, m.p., 107-109°C., yield 80%.

Analysis calculated for $C_{29}H_{39}ClF_{11}N_3O_3.H_2O$
Theory: C, 47.06; H, 5.58; N, 5.68;
Found: C, 47.28; H, 5.34; N, 5.87.

EXAMPLE 91

2'-Chloro-4'-nitro-1,2,2,3,3,4,4,5,5,6,6-undecafluorocyclohexanecarboxanilide, tetra-n-pentylammonium salt, m.p., 68-70°C., yield 88%.
Analysis calculated for $C_{33}H_{47}ClF_{11}N_3O_3.H_2O$
Theory: C, 49.78; H, 6.20; N, 5.28;
Found: C, 52.59; H, 7.29; N, 5.52.

EXAMPLE 92

2'-Chloro-4'-nitro-1,2,2,3,3,4,4,5,5,6,6-undecafluorocyclohexanecarboxanilide, tetra-n-hexylammonium salt, an oil, yield 64%.
Analysis calculated for $C_{37}H_{55}ClF_{11}N_3O_3.H_2O$
Theory: C, 52.51; H, 6.07; N, 4.97;
Found: C, 51.88; H, 6.98; N, 4.77.

EXAMPLE 93

2'-Chloro-4'-nitro-1,2,2,3,3,4,4,5,5,6,6-undecafluorocyclohexanecarboxanilide, tetra-n-heptylammonium salt, an oil, yield 40%.
Analysis calculated for $C_{41}H_{63}ClF_{11}N_3O_3.H_2O$
Theory: C, 54.21; H, 7.21; N, 4.63;
Found: C, 54.14; H, 7.39; N, 4.72.

EXAMPLE 94

2'-Chloro-4'-nitro-1,2,2,3,3,4,4,5,5,6,6-undecafluorocyclohexanecarboxanilide, hexadecyltrimethylammonium salt, m.p. 58-60°C., yield 50%.
Analysis calculated for $C_{32}H_{56}ClF_{11}N_3O_3.H_2O$
Theory: C, 49.14; H, 6.06; N, 5.37;
Found: C, 49.41; H, 5.85; N, 5.44.

EXAMPLE 95

2'-Chloro-4'-nitro-1,2,2,3,3,4,4,5,5,6,6-undecafluorocyclohexanecarboxanilide, octadecyltrimethylammonium salt, m.p., 57-59°C., yield 60%.
Analysis calculated for $C_{34}H_{49}ClF_{11}N_3O_3.H_2O$
Theory: C, 50.43; H, 6.30; N, 5.19;
Found: C, 52.35; H, 7.50; N, 5.11.

EXAMPLE 96

2'-Chloro-4'-nitro-1,2,2,3,3,4,4,5,5,6,6-undecafluorocyclohexanecarboxanilide, methyltris-($C_8$-$C_{10}$) ammonium salt, an oil, yield 26%.

EXAMPLE 97

2'-Chloro-4'-nitro-1,2,2,3,3,4,4,5,5,6,6-undecafluorocyclohexanecarboxanilide, methyltri-n-octylammonium salt, an oil, yield 48%.
Analysis calculated for $C_{38}H_{57}ClF_{11}N_3O_3.H_2O$
Theory: C, 52.72; H, 6.82; N, 4.86;
Found: C, 55.83; H, 7.45; N, 4.30.

EXAMPLE 98

2',5'-dichloro-4'-nitro-1,2,2,3,3,4,4,5,5,6,6-undecafluorocyclohexanecarboxanilide, tetra-n-propylammonium salt, m.p., 124-125°C., yield 91%.
Analysis calculated for $C_{25}H_{30}OCl_2F_{11}N_3O_3.H_2O$
Theory: C, 41.80; H, 4.49; N, 5.85;
Found: C, 42.03; H, 4.21; N, 5.86.

EXAMPLE 99

2',4'-Dinitro-1,2,2,3,3,4,4,5,5,6,6-undecafluorocyclohexanecarboxanilide, tetra-n-propylammonium salt, an oil, yield 63%.

Analysis calculated for $C_{25}H_{31}F_{11}N_4O_5.H_2O$
Theory: C, 43.22; H, 4.76; N, 8.07;
Found: C, 43.51; H, 4.41; N, 8.34.

EXAMPLE 100

2'-(Trifluoromethyl)-4'-nitro-1,2,2,3,3,4,4,5,5,6,6-undecafluorocyclohexanecarboxanilide, tetra-n-propylammonium salt, an oil, yield 21%.

EXAMPLE 101

2'-Cyano-4'-nitro-1,2,2,3,3,4,4,5,5,6,6-undecafluorocyclohexanecarboxanilide, tetra-n-propylammonium salt, m.p., 110-112°C., yield 77%.
Analysis calculated for $C_{26}H_{31}F_{11}N_4O_3.H_2O$
Theory: C, 46.29; H, 4.89; N, 8.31;
Found: C, 47.47; H, 4.73; N, 8.51.
This microanalysis indicated that the product was a mixture of parent and salt.

EXAMPLE 102

2'-Methyl-4'-nitro-1,2,2,3,3,4,4,5,5,6,6-undecafluorocyclohexanecarboxanilide, tetra-n-propylammonium salt, yield 34%.
Analysis calculated for $C_{26}H_{33}ClF_{11}N_3O_3.H_2O$
Theory: C, 44.74; H, 5.05; N, 6.02;
Found: C, 43.95; H, 4.23; N, 5.70.

EXAMPLE 103

2'-Cyano-4'-chloro-1,2,2,3,3,4,4,5,5,6,6-undecafluorocyclohexanecarboxanilide, hexadecyltrimethylammonium salt, m.p., 100-102°C., yield 90%.
Analysis calculated for $C_{33}H_{45}ClF_{11}N_3O.H_2O$
Theory: C, 52.00; H, 6.22; N, 5.51;
Found: C, 52.98; H, 5.75; N, 5.41.

EXAMPLE 104

2',4'-Dinitro-1,2,2,3,3,4,4,5,5,6,6-undecafluorocyclohexanecarboxanilide, hexadecyltrimethylammonium salt, an oil, yield 40%.
Analysis calculated for $C_{32}H_{45}F_{11}N_4O_5.H_2O$
Theory: C, 48.48; H, 5.98; N, 7.07;
Found: C, 48.27; H, 5.79; N, 6.86.

EXAMPLE 105

2',4',5'-Trichloro-1,2,2,3,3,4,4,5,5,6,6-undecafluorocyclohexanecarboxanilide, hexadecyltrimethylammonium salt, m.p., 85-88°C., yield 4%.
Analysis calculated for $C_{32}H_{44}Cl_3F_{11}N_2O.H_2O$
Theory: C, 47.68; H, 5.75; N, 3.48;
Found: C, 47.44; H, 5.59; N, 3.29.

EXAMPLE 106

2',4',6'-Trichloro-1,2,2,3,3,4,4,5,5,6,6-undecafluorocyclohexanecarboxanilide, hexadecyltrimethylammonium salt, an oil, yield 6%.
Analysis calculated for $C_{32}H_{44}Cl_3F_{11}N_2O.H_2O$
Theory: C, 47.68; H, 5.75; N, 3.48;
Found: C, 47.43; H, 5.82; N, 3.44.

EXAMPLE 107

2'-Methyl-4'-nitro-5'-chloro-1,2,2,3,3,4,4,5,5,6,6-undecafluorocyclohexanecarboxanilide, hexadecyltrimethylammonium salt, m.p., 75-77°C., yield 66%.
Analysis calculated for $C_{33}H_{47}ClF_{11}N_3O_3.H_2O$
Theory: C, 49.78; H, 6.20; N, 5.28;
Found: C, 49.56; H, 5.90; N, 5.25.

EXAMPLE 108

2',3',4',5'-Tetrachloro-1,2,2,3,3,4,4,5,5,6,6-undecafluorocyclohexanecarboxanilide, octadecyltrimethylammonium salt, m.p., 65-68°C., yield 17%.

Analysis calculated for $C_{34}H_{47}Cl_4F_{11}N_2O.H_2O$
Theory: C, 47.02; H, 5.69; N, 3.23;
Found: C, 44.90; H, 5.16; N, 3.18.

EXAMPLE 109

2'-Bromo-4'-nitro-1,2,2,3,3,4,4,5,5,6,6-undecafluorocyclohexanecarboxanilide, tri-n-butylammonium salt, m.p., 74-77°C., yield 63%.

Analysis calculated for $C_{22}H_{30}BrF_{11}N_3O_3$
Theory: C, 42.25; H, 4.37; N, 5.91;
Found: C, 41.95; H, 4.24; N, 5.80.

EXAMPLE 110

Mixture of 2'-bromo-4'-nitro1,2,2,3,3,4,4, 5,5,6,6-undecafluorocyclohexanecarboxanilide, and 2'-bromo-4'-nitro-x-(trifluoromethyl)-x,x,x,x,x,x,x,x-octafluorocyclopentanecarboxanilide

2'-Bromo-4'-nitro-1,2,2,3,3,4,4,5,5,6,6-undecafluorocyclohexanecarboxanilide was prepared from 1,2,2,3,3,4,4,5,5,6,6-undecafluorocyclohexanecarbonyl fluoride and 2-bromo-4-nitroaniline and recrystallized from toluene. The toluene mother liquor was evaporated to half the original volume. Product crystallized and was separated. The toluene mother liquor was chromatographed on silica gel with toluene, then recrystallized from toluene, m.p., 85-92°C., yield 19%.

Calculated for $C_{13}H_4BrF_{11}N_2O_3$
Theory: C, 29.74; H, 0.77; N, 5.34;
Found: C, 29.67; H, 0.60; N, 5.48.

$F^{19}$-NMR indicated that the product consisted of approximately 66% 2'-bromo-4'-nitro-1,2,2,3,3,4,4,5,5,6,6-undecafluorocyclohexanecarboxanilide, and approximately 33% 2'-bromo-4'-nitro-x-(trifluoromethyl)-x,x,x,x,x,x,x,x-octafluorocyclope tanecarboxanilide.

Yet other representative compounds are listed below. Unless indicated otherwise, each was prepared by essentially the same procedures as in Example 1 or in Examples 74-75.

EXAMPLE 111

2'-Nitro-4'-carboethoxy-1,2,2,3,3,4,4,5,5,6,6-undecafluorocyclohexanecarboxanilide, m.p., 74-76°C.

Analysis calculated for $C_{16}H_9F_{11}N_2O_5$
Theory: C, 37.06; H, 1.74; N, 5.40;
Found: C, 36.66; H, 1.78; N, 5.29.

EXAMPLE 112

2'-Bromo-4'-(methylsulfonyl)-1,2,2,3,3,4,4,5,5,6,6-undecafluorocyclohexanecarboxanilide, m.p., 150-152°C., yield 20%.

Analysis calculated for $C_{14}H_7BrF_{11}NO_3S$
Theory: C, 30.13; H, 1.26; N, 2.51;
Found: C, 30.04; H, 1.32; N, 2.50.

EXAMPLE 113

2'-Chloro-5'-(fluorosulfonyl)-1,2,2,3,3,4,4,5,5,6,6-undecafluorocyclohexanecarboxanilide, m.p., 107-108°C., yield 30%.

Analysis calculated for $C_{13}H_4ClF_{12}NO_3S$
Theory: C, 30.17; H, 1.14; N, 2.71;
Found: C, 30.16; H, 1.01; N, 2.78.

EXAMPLE 114

N-(2-Bromo-4-nitro-1-naphthyl)-1,2,2,3,3,4,4,5,5,6,6-undecafluorocyclohexanecarboxamide, m.p., 152-154°C., yield 20%.

Analysis calculated for $C_{17}H_6BrF_{11}N_2O_3$
Theory: C, 35.50; H, 1.05; N, 4.87;
Found: C, 35.26; H, 1.06; N, 4.76.

EXAMPLE 115

N-(4-Nitro-1-naphthyl)-1,2,2,3,3,4,4,5,5,6,6-undecafluorocyclohexanecarboxamide, m.p., 155-156°C., yield 28%.
Analysis calculated for $C_{17}H_7F_{11}N_2O_3$
Theory: C, 41.15; H, 1.42; N, 5.65;
Found: C, 40.99; H, 1.21; N, 5.58.

EXAMPLE 116

2'-Bromo-4'-nitro-6'-cyano-1,2,2,3,3,4,4,5,56,6-undecafluorocyclohexanecarboxanilide, m.p., 144-145°C., yield 13%.
Analysis calculated for $C_{14}H_3BrF_{11}N_3O_3$
Theory: C, 30.57; H, 0.55; N, 7.64;
Found: C, 30.65; H, 0.76; N, 7.43.

EXAMPLE 117

2'-Bromo-4'-carbomethoxy-1,2,2,3,3,4,4,5,5,6,6-undecafluorocyclohexanecarboxanilide, m.p., 128-131° C., yield 17%.
Analysis calculated for $C_{15}H_7BrF_{11}NO_3$
Theory: C, 33.48; H, 1.31; N, 2.60;
Found: C, 33.49; H, 1.41; N, 2.90.

EXAMPLE 118

2'-Methyl-3'-nitro-1,2,2,3,3,4,4,5,5,6,6-undecafluorocyclohexanecarboxanilide, m.p., 109-110°C., yield 43%.
Analysis calculated for $C_{14}H_7F_{11}N_2O_3$
Theory: C, 36.54; H, 1.53; N, 6.09;
Found: C, 36.25; H, 1.75; N, 5.99.

EXAMPLE 119

2'-Bromo-4'-chloro-6'-cyano-1,2,2,3,3,4,4,5,5,6,6-undecafluorocyclohexanecarboxanilide, m.p., 113-115°C., yield 6%.
Analysis calculated for $C_{14}H_3BrClF_{11}N_2O$
Theory: C, 31.17; H, 0.56; N, 5.19;
Found: C, 31.28; H, 0.63; N, 5.31.

EXAMPLE 120

2'-Chloro-4'-nitro-6'-cyano-1,2,2,3,3,4,4,5,5,6,6-undecafluorocyclohexanecarboxanilide, m.p., 145-147°C., yield 18%.
Analysis calculated for $C_{14}H_3ClF_{11}N_3O_3$
Theory: C, 33.26; H, 0.60; N, 8.31;
Found: C, 33.27; H, 0.77; N, 8.08.

EXAMPLE 121

2'-Bromo-4'-cyano-1,2,2,3,3,4,4,5,5,6,6-undecafluorocyclohexanecarboxanilide, m.p., 140-143°C., yield 40%.
Analysis calculated for $C_{14}H_4BrF_{11}N_2O$
Theory: C, 33.29; H, 0.80; N, 5.55;
Found: C, 33,49; H, 0.83; N, 5.26.

EXAMPLE 122

2',4'-Dichloro-6'-cyano-1,2,2,3,3,4,4,5,5,6,6-undecafluorocyclohexanecarboxanilide, m.p., 90-93°C., yield 4.1%.
Analysis calculated for $C_{14}H_3Cl_2F_{11}N_2O$
Theory: C, 33.97; H, 0.61; N, 5.66
Found: C, 34.04; H, 0.66; N, 5.42.

EXAMPLE 123

2'-Bromo-4'-(trifluoromethyl)-1,2,2,3,3,4,4,5,5,6,6-undecafluorocyclohexanecarboxanilide, m.p., 79-80°C., yield 19%.
Analysis calculated for $C_{14}H_4BrF_{14}NO$
Theory: C, 30.68; H, 0.74; N, 2.56;
Found: C, 30.73; H, 0.88; N, 2.43.

EXAMPLE 124

2',6'-Dichloro-4'-iodo-1,2,2,3,3,4,4,5,5,6,6-undecafluorocyclohexanecarboxanilide, m.p., 166°C., yield 65%.
Analysis calculated for $C_{13}H_3Cl_2F_{11}INO$
Theory: C, 26.20; H, 0.51; N, 2.35;
Found: C, 26.54; H, 0.00; N, 2.30.

EXAMPLE 125

2',6'-Dichloro-4'-(trifluoromethyl)-1,2,2,3,3,4,4,5,5,6,6-undecafluorocyclohexanecarboxanilide, m.p., 175-176°C., yield 40%.
Analysis calculated for $C_{14}H_3Cl_2F_{14}NO$
Theory: C, 31.25; H, 0.56; N, 2.60;
Found: C, 31.54; H, 0.83; N, 2.59.

EXAMPLE 126

2',3'-Dinitro-1,2,2,3,3,4,4,5,5,6,6-undecafluorocyclohexanecarboxanilide, m.p., 146-147°C., yield 11%.
Analysis calculated for $C_{13}H_4F_{11}N_3O_5$
Theory: C, 31.79; H, 0.82; N, 8.55;
Found: C, 31.93; H, 0.82; N, 8.68.

EXAMPLE 127

2'-Nitro-5'-methyl-1,2,2,3,3,4,4,5,5,6,6-undecafluorocyclohexanecarboxanilide, m.p., 85-86°C., yield 20%.
Analysis calculated for $C_{14}H_6F_{11}N_2O_3$
Theory: C, 36.62; H, 1.32; N, 6.10;
Found: C, 36.46; H, 1.35; N, 6.13.

EXAMPLE 128

2',4'-Dinitro-5'-(methylthio)-1,2,2,3,3,4,4,5,5,6,6-undecafluorocyclohexanecarboxanilide.
Sodium (0.2 gram; 0.008 mole) was added to 25 ml of ethanol. Methanethiol (0.5 gram; 0.008 mole) was then bubbled in, after which the compound of Example 55, 2' ,4'-dinitro-5'-fluoro-1,2,2,3,3,4,4,5,5,6,6-undecafluorocyclohexanecarboxanilide (2.0 grams; 0.004 mole), was added portionwise with stirring. The reaction mixture was stirred at room temperature for two more days. The reaction mixture was then worked up. Aqueous HCl/ice was added. The product precipitated and was separated by filtration, air dried, and recrystallized from toluene, m.p., 166-168°C., yield 51%.
Analysis calculated for $C_{14}H_6F_{11}N_3O_5S$
Theory: C, 31.30; H, 1.13; N, 7.82;
Found: C, 31.58; H, 1.24; N, 8.08.

EXAMPLE 129

2',4'-Dinitro-5'-ethoxy-1,2,2,3,3,4,4,5,5,6,6-undecafluorocyclohexanecarboxanilide.
This compound was prepared by reacting the compound of Example 55, 2',4'-dinitro-5'-fluoro-1,2,2,3,3,4,4,5,5,6,6-undecafluorocyclohexanecarboxanilide, with sodium ethoxide. The reaction and workup were carried out in the same manner described in the preceding example. The product initially melted at 73-85°C., but was recrystallized from ethanol and then melted at 105°C., yield 32%.
Analysis calculated for $C_{15}H_8F_{11}N_3O_5$
Theory: C, 34.70; H, 1.55; N, 8.09;
Found: C, 34.51; H, 1.50; N, 7.92.

EXAMPLE 130

2',6'-Dinitro-4'-bromo-1,2,2,3,3,4,4,5,5,6,6-undecafluorocyclohexanecarboxanilide, m.p., 130-132°C., yield 7%.
Analysis calculated for $C_{13}H_3BrF_{11}N_3O_5$
Theory: C, 27.36; H, 0.53; N, 7.36;
Found: C, 27.68; H, 0.78; N, 7.21.

EXAMPLE 131

2',6'-Dichloro-4'-cyano-1,2,2,3,3,4,4,5,5,6,6-undecafluorocyclohexanecarboxanilide, m.p., 220-221°C., yield 41%.
Analysis calculated for $C_{14}H_3Cl_2F_{11}N_2O$
Theory: C, 33.97; H, 0.61; N, 5.66;
Found: C, 34.21; H, 0.81; N, 5.68.

EXAMPLE 132

2'-Nitro-5'-chloro-1,2,2,3,3,4,4,5,5,6,6-undecafluorocyclohexanecarboxanilide, m.p., 123-124°C., yield 35%.
Analysis calculated for $C_{13}H_4ClF_{11}N_2O_3$
Theory: C, 32.49; H, 0.84; N, 5.83;
Found: C, 32.29; H, 0.91; N, 5.88.

EXAMPLE 133

2',6'-Dichloro-4'-bromo-1,2,2,3,3,4,4,5,5,6,6-undecafluorocyclohexanecarboxanilide, m.p., 169-170°C., yield 50%.
Analysis calculated for $C_{13}H_3BrCl_2F_{11}NO$
Theory: C, 28.44; H, 0.55; N, 2.55;
Found: C, 28.70; H, 0.72; N, 2.52.

EXAMPLE 134

2',4'-Dichloro-6'-bromo-1,2,2,3,3,4,4,5,5,6,6-undecafluorocyclohexanecarboxanilide, m.p., 176-177°C., yield 37%.
Analysis calculated for $C_{13}H_3BrCl_2F_{11}NO$
Theory: C, 28.44; H, 0.55; N, 2.55;
Found: C, 28.55; H, 0.75; N, 2.81.

EXAMPLE 135

2',4'-Dichloro-6'-iodo-1,2,2,3,3,4,4,5,5,6,6-undecafluorocyclohexanecarboxanilide, m.p., 164-166°C., yield 33%.
Analysis calculated for $C_{13}H_3Cl_2F_{11}INO$
Theory: C, 26.20; H, 0.51; N, 2.35;
Found: C, 26.44; H, 0.75; N, 2.19.

EXAMPLE 136

2'-Bromo-4'-nitro-1,1,2,3,3,4,4,4a,5,5,6,67,7,8,8,8a-heptadecafluoro-1,2,3,4,4a,5,6,7,8,8a-decahydro-2-naphthalenecarboxan ilide, m.p., 62-80°C., yield 34%.
Analysis calculated for $C_{17}H_4BrF_{17}N_2O_3$
Theory: C, 29.72; H, 0.59; N, 4.08;
Found: C, 29.46; H, 0.74; N, 3.97.

EXAMPLE 137

2',4'-Dinitro-1,1,2,3,3,4,4,4a,5,5,6,6,7,7,8,8,8a-heptadecafluoro-1,2,3,4,4a,5,6,7,8,8a-decahydro-2-naphthalenecarboxanilid e, an oil, yield 35%.
Analysis calculated for $C_{17}H_4F_{17}N_3O_5$
Theory: C, 31.26; H, 0.62; N, 6.43;
Found: C, 31.03; H, 0.80; N, 6.41.

EXAMPLE 138

2',4'-Dinitro-5'-fluoro-1,1,2,3,3,4,4,4a,5,56,6,7,7,8,8,8a-heptadecafluoro-1,2,3,4,4a,5,6,7,8,8a-decahydro-2-naphthaleneca rboxanilide, an oil, yield 26%.
Analysis calculated for $C_{17}H_3F_{18}N_3O_5$
Theory: C, 30.42; H, 0.45; N, 6.26;
Found: C, 30.57; H, 0.60; N, 6.41.

EXAMPLE 139

2'-Bromo-4'-nitro-3-(trifluoromethyl)-1,2,2,3,4,4,5,5,6,6-decafluorocyclohexanecarboxanilide.
In a Teflon jar equipped with a stainless steel condensor maintained at from -40 to -50°C., approximately 130 cc of commercial anhydrous HF underwent a pre-electrolysis to remove the last traces of water. An electrode pack of about 2 $in^3$ in size consisting of alternating nickel and carbon steel plates was used under a nitrogen atmosphere at a maximum current density of about 20 $ma/cm^2$ and at or below a cell voltage of 5.2 volts relative to a copper reference electrode. 4.2 g (0.02 moles) of distilled m-(trifluoromethyl)benzoyl chloride was added, and 5.6 amp hrs was passed (75% of theoretical). The reaction mixture was extracted with three 20 cc portions of $CFCl_3$, and the extracts were added to 3.2 g of 2-bromo-4-nitro-aniline (0.015 moles) and 2.5 g (0.025 moles) of triethylamine in 25 cc methylene chloride. The organic layer was washed with dilute HCl, dried over sodium sulfate, and chromatographed on silica gel with toluene to give 2'-bromo-4'-nitro-3-(trifluoromethyl)-1,2,2,3,4,4,5,5,6,6-decafluorocyclohexanecarboxanilide, m.p., 55-60°C., yield 35% from acid chloride, (after recrystallization from toluene, m.p., 79-82°C.). $F^{19}$ NMR was consistent with a mixture of cis/trans isomers. A similar procedure was used in preparing each of Examples 140-143 and 145.
Analysis calculated for $C_{14}H_4BrF_{13}N_2O_3$
Theory: C, 29.24; H, 0.70; N, 4,87;
Found: C, 29.20; H, 0.83; N, 5.16;
Found after recrystalization:
C, 29.14; H, 0.66; N, 4.77.
A 4% yield of the same product, likewise a mixture of cis/trans isomers, was obtained using m-toluoyl chloride in the above reaction, by separating the product acid fluoride from HF without the benefit of extraction.
Found: C, 29.36; H, 0.80; N, 4.79.

EXAMPLE 140

2'-Bromo-4'-nitro-4-(trifluoromethoxy)-1,2,2,3,3,4,5,5,6,6-decaflurocyclohexanecarboxanilide, m.p., 90-92°C., yield 20% from p-(trifluoromethoxy)benzoyl chloride (after recrystallization from toluene, m.p., 101-104°C.).
Analysis calculated for $C_{14}H_4BrF_{13}N_2O_3$
Theory: C, 28.45; H, 0.68; N, 4.74;
Found: C, 28.64; H, 0.69; N, 4.47;
Found after recrystallization:
C, 28.42; H, 0.73; N, 4.64.

EXAMPLE 141

2'-Bromo-4'-nitro-4-(pentafluoroethoxy)-1,2,2,3,3,4,5,5,6,6-decafluorocyclohexanecarboxanilide, m.p., 75-91°C., yield 5% from p-ethoxybenzoyl chloride. After recrystallization from toluene, the product melted at 128-133°C.
Analysis calculated for $C_{15}H_4BrF_{15}N_2O_3$
Theory: C, 28.08; H, 0.62; n, 4.37;
Found after recrystallization:
C, 28.38; H, 0.75; N, 4.66.

EXAMPLE 142

50:50 Mixture of 2'-bromo-4'-nitro-4-chloro1,2,2,3,3,4,5,5,6,6-decafluorocyclohexanecarboxanilide and 2'-bromo-4'-nitro-1,2,2,3,3,4,4,5,5,6,6-undecafluorocyclohexanecarboxan lide, m.p., 78-84°C., yield 11% from p-chlorobenzoyl chloride.
Analysis calculated for $C_{13}H_4BrClF_{10}N_2O_3$
Theory: C, 28.83; H, 0.74; N, 5.17;
Found: C, 29.15; H, 0.93; N, 4.86.

EXAMPLE 143

Mixture of two isomers of 2'-bromo-4'-nitro-x,x-dichloro-x,x,x,x,x,x,x,x,x,-nonofluorocyclohexanecarboxanilide, 2'-bromo-4'-nitro-x-chloro-x,x,x,x,x,x,xx,x,x-decafluorocyclohexanecarb xanilide, and 2'-bromo-4'-nitro-1,2,2,3,3,4,4,5,5,6,6-undecafluorocyclohexanecarboxan lide, an oil, in the ratio of 22:33$_1$/$_3$:33$_1$/$_3$:11. The yield was 6% from 2,4-dichlorobenzoyl chloride.

EXAMPLE 144

2,4'-Dinitro-1,2,2,3,3,4,4,5,5,6,6-undecafluorocyclohexanecarboxanilide, dimethylbis ($C_{10}$-$C_{18}$) ammonium salt, an oil, yield 92%.

EXAMPLE 145

50:50 Mixture of 2'-bromo-4'-nitro-2-(trifluoromethyl)-1,2,3,3,4,4,5,5,6,6-decafluorocyclohexanecarboxanilide and 2'-bromo-4'-nitro-x,x-bis(trifluoromethyl)-x,x,x,x,x,x-heptafluorocyclopentanecarboxanilide, m.p., 66-80°C., yield 5.1% from o-(trifluoromethyl)benzoyl chloride.

Analysis calculated for $C_{14}H_4BrF_{13}N_2O_3$

Theory: C, 29.24; H, 0.70; N, 4.87;
Found: C, 29.41; H, 0.83; N, 4.67.

EXAMPLE 146

2'-Bromo-4'-nitro-1,2,2,3,3,4,4,5,5,6,6-undecafluorocyclohexanecarboxanilide.

This compound, which is also the subject of Example 1, was prepared in an alternate procedure employing p-nitroaniline as starting material. To a 100 ml round bottom flask equipped with a reflux condenser, stirrer, thermometer, and addition funnel were added 37 ml of methylene chloride and 11.86 g of pyridine. Keeping the reactor contents under 40°C with a water bath, 25.17 g of bromine were added over 3 to 5 minutes and the solution was stirred 30 minutes at 20-25°C.

50 ml of methylene chloride and 20.72 g of p-nitroaniline were placed in a separate 250 ml flask equipped with an addition funnel, condenser, thermometer, and mechanical stirrer. Keeping the temperature below 30°C with a water bath, the methylene chloride solution of pyridine/bromine prepared above was added dropwise to the slurry of p-nitroaniline in methylene chloride over 10 minutes. 20 ml of additional methylene chloride was used to transfer the pyridine/bromine solution. The reaction mixture was stirred for 30 minutes at 25°C.

The reaction mixture was then cooled in an ice bath to 0-5°C and 15.18 g of triethylamine were added all at once. Next, 82.03 g of perfluorocyclohexanecarbonyl fluoride were added dropwise over 15 minutes, maintaining the temperature below 20°C., and the reaction mixture was stirred for 30 minutes at 5-10°C.

The reaction mixture was then worked up. First 100 ml of aqueous acetic acid solution (100 ml of water and 10 ml of acetic acid) were added and the reaction mixture was agitated for 10 minutes (pH 4.3). The layers were then separated. The aqueous layer was extracted with three 40-ml portions of methylene chloride. The methylene chloride layers were combined, washed with 110 ml of water, and concentrated to an oily residue by distilling to 80°C. pot temperature. 300 ml of methanol was added and 170 ml of mixed solvent distilled off. Maintaining reflux, 50 ml of water were added as rapidly as possible.

The residue was then cooled slowly to 0°C. with agitation. The product precipitated and was removed by filtration, washed with two 60-ml portions of cold methanol water (3:1 volume/volume), and dried, yield 69.75 g (86.3%).

EXAMPLE 147

2'-Nitro-4'-thiccyanato-1,2,2,3,3,4,4,5,5-undecafluorocyclohexanecarboxanilide, m.p., 81-83°C.

EXAMPLE 148

2',5'-Dichloro-4'-bromo-1,2,2,3,3,4,4,5,5,6,6-undecafluorocyclohexanecarboxanilide, m.p., 102-104°C, yield 46%.

Utility

As noted above, the compounds of the present invention exhibit excellent insecticidal and arachnicidal activity. This activity is illustrated by the following tests.

Representative compounds of the invention were tested and found to be highly active against a variety of insects and arachnids, including:

mosquito larve - Aedesaegypti
Mexican bean beetle larve - Epilanchnavarivestis

Southern armyworm larve - Spodopteraeridania
two spotted spider mite - Tetranychusurticae
melon aphid - Aphisgossypii
housefly - Muscadomestica
milkweed bugs - oncopeltisfasciatus
oriental cockroach - Blattaorientalis
cotton boll weevil - Anthonomusgrandis
Southern corn rootworm larve - Diabroticaundecimpunctata howardi
cotton bollworm - Heliothiszea
beet armyworm - Spodopteraexigua
corn leafhopper - Dalbulusmaidis
white grub - Phyllophaga spp.
black cutworm - Agrotisipsilon
onion fly - Deliaantiqua
cabbage root fly - Deliabrassicae
turnip cutworm - Agrotissegetum
wireworm - Agriotes sp.
cereal aphid - Rhopalosiphumpadi
cotton leafworm - Spodopteralittoralis
diamondback moth - Plutellaxylostella
German cockroach - Blattellagermanica

The compounds of the present invention are effective against organisms that attack foliage as well as organisms that live in the soil and attack roots and other underground parts of plants.

Therefore, in another embodiment, the present invention is directed to a method of inactivating an insect or arachnid which comprises applying to a locus of the insect or arachnid an effective amount of one or more of the compounds of the present invention.

Insects and arachnids against which the present method can be practiced include the various species identified above as well as many others, including, among the insects, the following

Coleoptera

Anthonomusgrandis - boll weevil
Conotrachelusnenuphar - plum curculio
Curculiocaryae - pecan weevil
Diabrotica spp. - rootworms and cucumber beetles
Echinocnemussquameus - rice plant weevil
Epitrixhirtipennis - tobacco flea beetle
Eutheolahumilisrugiceps - sugarcane beetle
Hyperapostica - alfalfa weevil
Leptinotarsadecimlineata - Colorado potato beetle
Lissorhoptrusoryzophilus - rice water weevil
Oulemaoryzae - rice leaf beetle
Phyllotretastriolata - striped flea beetle
Melanotus spp. and Agriotis spp. - wireworms
Stenolophuslecontei - seedcorn beetle
Popilliajaponica - Japanese beetle
Sphenophorusmaidis - maize billbug
Systenablanda - palestriped flea beetle

Diptera

Contariniasorghicola - sorghum midge
Dacusdorsalis - oriental fruit fly
Liriomyza spp. - leaf miner
Rhagoletispomonella - apple maggot
Hylemia spp. and Delia spp. - root and seed maggots

Heteroptera

Anasatristis - squash bug
Blissusleucopterusleucopterus - chinch bug
Euschistusservus - brown stink bug
Lygus lineolaris - tarnished plant bug
Nezaraviridula - southern green stink bug
Oebaluspugnax - rice stink bug

Pseudatomoscelisseriatus - cotton fleahopper

Homoptera

Clastopteraachatina - pecan spittlebug
Empoascafabae - potato leafhopper
Eriosomalanigerum - wooly apple aphid
Fioriniatheae - tea scale
Graminellanigrifrons - blackfaced leafhopper
Iceryapurchasi - cottony cushion scale
Laodelphaxstriatellus - small brown planthopper
Lepidosaphesulmi - oystershell scale
Myzuspersicae - green peach aphid
Nephotettixcincticeps - green rice leafhopper
Niloparvatalugens - brown rice planthopper
Phylloxeradevastatrix - pecan phylloxera
Planococcuscitri - citrus mealybug
Psyllapyricola - pear psylla
Quadraspidiotusperniciosus - San Jose scale
Rhopalosiphummaidis - corn leaf aphid
Siphaflava - yellow sugarcane aphid
Sogatellafurcifera - whitebacked planthopper
Spissistilusfestinus - threecornered alfafa hopper
Trialeurodesvaporariorum - greenhouse whitefly
Anuraphismaidiradicis - corn root aphid

Hymenoptera

Atta spp. - leafcutter ants
Camponotus spp. - carpenter ants
Dolichovespula spp. - yellowjackets
Solenopsisinvicta - red imported fire ant
Tetramoriumcaespitum - pavement ant
Vespidae spp. - hornets, wasps

Isoptera

Coptotermesformosanus - Formosan subterranean termite
Reticulitermesflavipes - eastern subterranean termite

Lepidoptera

Agrotis spp. and other genera - cutworms
Alabamaargillacea - cotton leafworm
Anticarsiagemmatalis - velvetbean caterpillar
Buccalatrixthurberiella - cotton leafperforator
Chilosuppressalis - rice stem borer
Choristoneurafumiferana - spruce budworm
Cydiapomonella - codling moth
Elasmopalpuslignosellus - lesser cornstalk borer
Grapholitamolesta - oriental fruitmoth
Heliothisvirescens - tobacco budworm
Heliothiszea - cotton bollworm
Keiferialycopersicella - tomato pinworm
Ostrinianubilalis - European corn borer
Parnaraguttata - rice skipper
Pectinophoragossypiella - pink bollworm
Pierisrapae - imported cabbageworm
Plutellaxylostella - diamondback moth
Pseudoplusiaincludens - soybean looper
Sesamiainferens - rice swarming caterpillar
Spodopteralittoralis - Egyptian cotton leafworm
Spodoptera spp. - armyworms
Synanthedon spp. - clearwing moths
Trichoplusiani - cabbage looper

Tryporyzaincertula - yellow rice borer
Crambus spp. - webworms

Orthoptera

Blatella spp. - cockroaches
Gryllus spp. - field crickets
Melanoplus spp. - grasshoppers
Periplaneta spp. - cockroaches
Scapteriscusacletus - southern mole cricket

Thysanoptera

Frankliniellatritici - flower thrips
Sericothripsvariabilis - soybean thrips
Thripssimplex - gladiolus thrips
Thripstabaci - onion thrips
and among the arachnids, the following:

| Family | Scientific Name | Common Name |
|---|---|---|
| ACARIDAE | | |
| | Aleurobius farinae | Flour mite |
| | Rhizoglyphus echinopus | Bulb mite |
| | Rhizoglyphus elongatus | Elongate mite |
| | Rhizoglyphus rhizophagus | Root mite |
| | Rhizoglyphus sagittatae | Balsam root mite |
| | Rhizoglyphus tarsalis | Beet mite |
| ERIOPHYIDAE | | |
| | Abacarus hystrix | |
| | Aceria brachytarsus | |
| | Aceria essigi | Redberry mite |
| | Aceria ficus | |
| | Aceria fraxinivorus | |
| | Aceria granati | |
| | Aceria parapopuli | Cottonwood mite |
| | Aceria sheldoni | Citrus bud mite |
| | Aceria tulipae | Wheat curl mite |
| | Aceria schlechtendali | Apple rust mite |
| | Eriophyes convolvens | |
| | Eriophyes insidiosus | |
| | Eriophyes malifoliae | Apple leaf mite |
| | Eriophyes padi | Plum twig gall mite |

| | | |
|---|---|---|
| | <u>Eriophyes</u> <u>pruni</u> | Plum leaf gall mite |
| | <u>Eriophyes</u> <u>pyri</u> | Pear leaf blister mite |
| | <u>Eriophyes</u> <u>ramosus</u> | Juniper mite |
| | <u>Eriophyes</u> <u>ribis</u> | Currant gall mite |
| | <u>Eriophyes</u> <u>vitis</u> | Grape erineum mite |
| | <u>Phyllocoptes</u> <u>gracilis</u> | Blackberry mite |
| | <u>Phyllocoptruta</u> <u>oleivora</u> | Citrus rust mite |
| | <u>Phytoptus</u> <u>ribis</u> | |
| | <u>Trisetacus</u> <u>pini</u> | Pine needle mite |
| | <u>Vasates</u> <u>amygdalina</u> | David peach mite |
| | <u>Vasates</u> <u>cornutus</u> | Peach silver mite |
| | <u>Vasates</u> <u>eurynotus</u> | Celery rust mite |
| | <u>Vasates</u> <u>schlechtendali</u> | Rusty leaf mite |
| EUPODIDAE | | |
| | <u>Linopodes</u> spp. | |
| PENTHALEIDAE | | |
| | <u>Halotydeus</u> <u>destrustor</u> | Redlegged earth mite |
| | | Black sand mite |
| | <u>Penthaleus</u> <u>major</u> | Winter grain mite |
| | | Blue oat mite |
| PYEMOTIDAE | | |
| | <u>Siteroptes</u> <u>cerealium</u> | |
| TARSONEMIDAE | | |
| | <u>Polyphagotarsonemus</u> <u>latus</u> | Broad mite |
| | <u>Steneotarsonemus</u> <u>pallidus</u> | Cyclamen mite |

TENUIPALPIDAE

| | |
|---|---|
| Brevipalpus californicus | California citrus mite |
| Brevipalpus obovatus | |
| Brevipalpus lewisi | Flat mite |
| Tenuipalpes granati | |
| Tenuipalpes pacificus | |

TETRANYCHIDAE

| | |
|---|---|
| Bryobia arborea | Brown mite |
| Bryobia rubrioculus | |
| Eotetranychus coryli | |
| Eotetranychus lewisi | Lewis spider mite |
| Eotetranychus sexmaculatus | Six spotted spider mite |
| Eotetranychus weldoni | Weldon's mite |
| Eotetranychus willametti | |
| Eutetranychus banksi | Texas citrus mite |
| Mediolata mali | Apple mite |
| Oligonychus ilicis | Southern red mite |
| Oligonychus pratensis | Banks grass mite |
| Oligonychus ununguis | Spruce tree spider mite |
| Panonychus citri | Citrus red mite |
| Panonychus ulmi | European red mite |
| Paratetranychus modestus | Corn mite |
| Paratetranychus pratensis | Date mite |
| Paratetranychus viridis | Green mite |

| | |
|---|---|
| _Petrobia decepta_ | Barley mite |
| _Schizotetranychus celarius_ | Bamboo mite |
| _Schizotetranychus pratensis_ | Alfalfa mite |
| _Tetranychus canadensis_ | Fourspotted mite |
| _Tetranychus cinnabarinus_ | Carmine mite |
| _Tetranychus mcdanieli_ | McDaniel mite |
| _Tetranychus pacificus_ | Pacific mite |
| _Tetranychus schoenei_ | Schoene mite |
| _Tetranychus telarius_ | Common red spider |
| _Tetranychus urticae_ | Twospotted spider mite |
| _Tetranychus turkestani_ | Strawberry mite |
| _Tetranychus desertorum_ | Desert mite |

The present compounds are employed for the control of insects and arachnids in accordance with standard practices of the agricultural chemical industry. Thus, the compounds, while they can be employed alone, are preferably formulated with conventional adjuvants, as described in more detail in the section below concerning Formulations. The amount of the present compounds which will provide insecticidal and/or arachnicidal activity is not critical and will vary widely, depending on the locus (foliage, soil, etc.), the susceptibility of the particular insect or arachnid, the level of activity of the particular compound chosen, weather and soil conditions, and the like. In general, when employing the compounds in spray formulations to be applied to foliage, concentrations of from 1 to 5000 ppm are efficacious. Lower concentrations of from 1 to 1000 ppm, and even from 1 to 100 ppm, often suffice. In the case of application to soil to control soil-dwelling organisms, concentrations in the soil of from 1 ppm or less to as much as 50 ppm provide good activity With the more active members of the series, lesser concentrations of from 1 to 10 ppm provide good activity. The compound of Example 1, as shown by the above examples, will provide control in the field at application rates of approximately 0.1 lb/acre (0.112 ka/ha) to 1.0 lb/acre (1.12 kg/ha).

The compounds to be employed in accordance with the present invention can be applied by standard agricultural equipment and techniques. However, the compounds exhibit some phytotoxicity, and this fact should be taken into account in selecting an application technique. For application to soil to control soil-dwelling organisms, it is preferred that the compounds be applied in a technique by which a pesticide is prevented from contacting the seeds, for example, post presswheel placement.

## Preferred Embodiments

A preferred embodiment of the present invention is the use of selected compounds for the control of corn rootworm. Those compounds exhibiting good activity against corn rootworm will be apparent from Table XIII above.

Among the compounds of the present invention, there are various preferences:

(1) a preference for $R^1$ = fluorocyclohexyl as defined,
(2) a preference for n = p = q = 0 or 1,
(3) a preference for $R^3$ = hydrogen,
(4) a preference for $R^4$ = substituted aryl as defined, and especially for the following substituted aryl groups:
(a) 2,4(or 2,5)-disubstituted phenyl of the formula

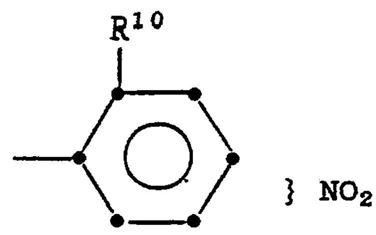

wherein $R^{10}$ = fluoro, chloro, bromo, iodo, cyano, methyl, trifluoromethyl, or nitro;
(b) 2,4-disubstituted phenyl of the formula

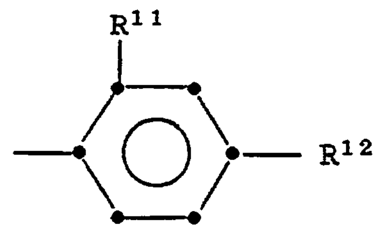

wherein $R^{11}$ = cyano or trifluoromethyl and $R^{12}$ = bromo or chloro;
(c) 2,4,5(or 2,4,6)-trisubstituted phenyl of the formula

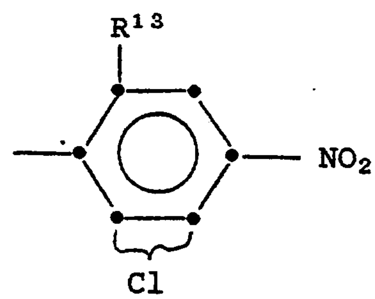

wherein $R^{13}$ = bromo, chloro, or methyl;
(d) 2,3,4,5-tetrachlorophenyl; and
(e) pentafluorophenyl.

Among the salts of the present invention, it has been found that the ammonium salts are preferred, and among the ammonium salts, the following particular salts are especially advantageous: hexadecyltrimethylammonium, octadecyltrimethylammonium, tributylammonium, tris(2-hydroxethyl)ammonium, tris(2 or 3-hydroxypropyl)ammonium, and dimethylbis($C_{10}$-$C_{18}$)ammonium.

Herbicidal Activity

In addition to exhibiting activity against insects and arachnids, the present compounds also exhibit some herbicidal activity. In general, the herbicidal activity is expressed at rates higher than those at which insecticidal and arachnicidal activity is exhibited. Therefore, the preferred insecticidal/arachnicidal utility of the present invention can generally be practiced with minimal or no phytotoxicity.

Representative compounds of the present invention were evaluated for herbicidal efficacy in standard screening tests. In these tests, each compound was evaluated against a number of crop and weed species, for both preemergent and postemergent effect.

Many of the compounds of the present invention exhibit herbicidal activity at rates of from 0.05 to 8 lbs/acre. Therefore, in another embodiment, the present invention is directed to a method for inhibiting the growth of a plant which comprises applying to the plant an effective amount of a compound of the present invention. The compounds can be applied pre-emergently for the control of germinating seeds or post-emergently for the control of existing vegetation. The compounds are preferably formulated as described below.

Other Utilities

Compounds of the present invention have also exhibited nematocidal, fungicidal, anthelmintic, and ectoparasiticidal activity. By appropriate selection of rates and methods of application, therefore, the present compounds can be employed for these other utilities. For all of these uses, the compounds are employed in conventional manners.

For example, the compound of Example 1 is effective in controlling various pathogenic nematode species, while having little effect on the benign Saprophagous nematodes. Thus, in another embodiment of the present invention, the compound of Example 1 or a salt thereof is applied to nematode infested soil in an effective, nematocidal amount. The method is practiced in accordance with standard techniques for the application of nematocides. In general, good nematocidal activity can be expected at rates of 1-10 lbs/acre. The compound can be formulated as described below in the Formulations section; in general, granular formulations are preferred.

Fungicidal Activity

Many of the compounds of the present invention exhibit useful fungicidal activity. Accordingly, in another embodiment, the present invention is directed to a method of inhibiting a plant pathogenic fungal organism which comprises applying to a locus of the organism an inhibiting amount of a compound of the present invention. The method is practiced in accordance with standard techniques for the use of fungicides. The compounds can be formulated as described below in the Formulations section. In general, good fungicidal efficacy can be expected at rates of 0.5-5.0 lbs/acre.

Preferred for this embodiment are compounds of four subgenera. A first subgenus is defined by the formula

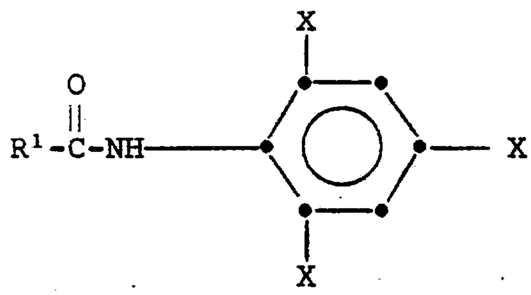

and includes salts thereof, as defined above. In the foregoing formula, each X represents halo (i.e., bromo, chloro, fluoro, or iodo), which is independently selected except that no more than one X can represent iodo. This subgenus is exemplified by Examples 57, 60, 106, 124, 133, 134, and 135.

A second group of compounds, also a subgenus of the generic scope described above, is of the following formula

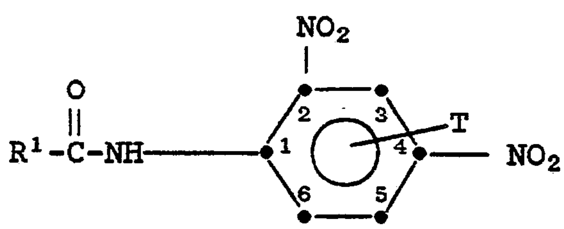

and includes salts thereof, as defined above. In the foregoing formula, T represents fluoro, $C_1$-$C_4$ alkoxy, or $C_1$-$C_4$ alkylthio, located at either the 3- or 5-position. This group of compounds is exemplified by Examples 55, 128, 129, and 138.

A third group of compounds preferred for antifungal use is again a subgenus of the compounds generically described above. This group is defined by the following formula

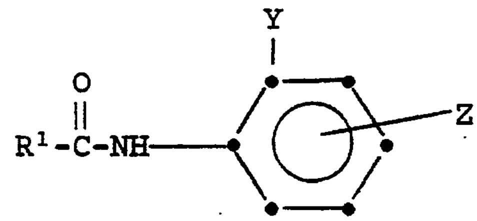

and includes salts thereof, as defined above. In the foregoing formula, Y represents halo, nitro, or methyl, and Z represents nitro or fluorosulfonyl. This group of compounds is exemplified by Examples 5, 8, 11, 29, 113, 118, and 126.

The fourth preferred group consists of compounds of formula (I) wherein $R^4$ is phenyl substituted with two groups, including a thiocyanato group and a nitro, $R^5$ or iodo group. This group of compounds is illustrated by Example 147.

Formulations

For any of their various uses, the compounds of the present invention are preferably formulated with a suitable agriculturally acceptable carrier. Typically such a formulation will contain from 0.05 to 95.0 percent by weight of the active ingredient. Examples of such compositions include sprayable formulations, such as wettable powders, aqueous suspensions and emulsifiable concentrates; and solid compositions, such as dusts, granules, and dry-flowable pellets. The compounds can also be formulated with fertilizer and applied to soil to achieve both an effect in accordance with the present invention as well as fertilization of the crop.

Sprayable formulations are in the form of concentrated compositions which can be diluted with water to form water dispersions or emulsions containing in the range from 0.05 percent to 10 percent of the active agent by weight. Such water dispersions or emulsions are sprayed onto plants or onto soil. The concentrated compositions may be either solids, usually known as wettable powders or dry flowables, or liquids, usually known as emulsifiable concentrates and aqueous suspensions.

A typical wettable powder comprises an intimate mixture of an active ingredient of the invention, an inert carrier, and surfactants. The concentration of the active agent is usually from 25 percent to 90 percent by weight. The inert carrier is usually chosen from among the attapulgite clays, the montmorillonite clays, the diatomaceous earths, the kaolinites, or the purified silicates. Effective surfactants, comprising from 0.5 percent to 10 percent by weight of the wettable powder, are chosen from among the condensed naphthalenesulfonates, the alkyl sulfates and the alkyl arylethoxylates. Suspending agents, such as the sulfonated lignins can also be added.

A typical emulsifiable concentrate comprises from 0.1 to 6 pounds (from 0.045 kg to 3.05 kg) of a compound of the invention per gallon of liquid, dissolved in a mixture of organic solvents and emulsifiers. The organic solvent is chosen with regard to its solvency and its cost. Useful solvents include the aromatics, especially the xylenes and the heavy aromatic naphthas. Hydrophilic cosolvents such as DMF, cyclohexanone, and the glycol ethers such as 2-methoxyethanol may be included. Other organic solvents may also be used, including the terpenic solvents and kerosene; methyl heptyl ketone and other high molecular weight ketones; cyclohexyl acetate and other high molecular weight esters. Suitable emulsifiers for emulsifiable concentrates are chosen from the alkylbenzenesulfonates, naphthalenesulfonates, and nonionic surfactants such as alkylphenol adducts of polyoxyethylene, and are used at similar percentages as for wettable powders.

An aqueous suspension, or flowable, is comprised of a finely ground suspension of the active ingredient dispersed in a water based system. This type of formulation is particularly useful for compounds with low water solubility. The concentration of active agent is usually from 15 to 60 percent by weight. A typical aqueous suspension may comprise wetting and dispersing agents, antifreeze components, thickening or bulking agents, as well as water and the active ingredient.

Dust compositions containing a compound of the present invention usually contain from 0.1 to 50 percent by weight of the compound. Dusts are prepared by intimately mixing and finely grinding the active agent with an inert solid such as ground montmorillonite clay, attapulgite clay, talc, ground volcanic rock, kaolin clay, or other inert, relatively dense, inexpensive substances.

Solid, granular compositions are convenient for the application of compounds of this invention to the soil and will contain the active agent in an amount from 0.1 to 25 percent by weight. Granules comprise a compound of the invention dispersed on a granular inert carrier such as coarsely ground clay of from 0.1 to 3 mm particle size. The active ingredient is most conveniently applied to the clay by dissolving it in an inexpensive solvent, such as acetone, methylene chloride, xylene or other petroleum solvents, methoxy propylene glycol, or the like, and applying the solution to the sized clay in an appropriate solids mixer. The solvent is then typically removed by evaporation; however removal is not essential. Alternatively, any of the present compounds which is an oil can be sprayed, with or without heating, directly onto clay.

Likewise, any of the present compounds which is a solid can be melted and then sprayed directly onto clay.

Numerous representative formulations of the compounds to be employed in accordance with the present invention were prepared. Examples follow.

EXAMPLE A

Emulsifiable Concentrate

A 1 lb/gallon (1 lb/gal = $1.198.10^2$ kg/$m^2$) emulsifiable concentrate was prepared by dissolving the compound of Example 1, estimated to be of 97% purity, in xylene and adding Toximul H and Toximul D (each of Toximul H and Toximul D is a sulfonate-nonionic blend of emulsifiers produced by Stepan Chemical Co.)

| Ingredient | Percent by Weight |
|---|---|
| Compound of Example 1 | 13.2 |
| Xylene | 81.8 |
| Toximul H | 3.0 |
| Toximul D | 2.0 |

EXAMPLE B

Wettable Powder

A 20% wettable powder of the compound of Example 1 was prepared by mixing the first five listed ingredients in conventional fashion. After these ingredients had been mixed, 3% of Selogen HR (a dispersing agent produced by Diamond Shamrock) and 3% of a mixture of

(1) 22% of TMN-6, a surfactant sold by Union Carbide, trimethylnonane/6 moles of ethylene oxide,
(2) 28% of Triton X-100®, a surfactant sold by Rohm & Haas, octylphenol/10 moles of ethylene oxide, and
(3) 50% of HiSil, a lightweight silica sold by Pittsburg Plate Glass Co.

were added and blended until uniform. The ingredients and the % of each in the final formulation were as follows.

| Ingredient | Percent by weight |
|---|---|
| Compound of Example 1, 95% purity | 21.06 |
| Stepanol ME, sodium lauryl sulfate from Stepan Chemical Co. | 4.72 |
| Polyfon O, a dispersant from Westvaco Corp., Polychemicals Dept. | 4.72 |
| Zeolex-7, a sodium silicoaluminate from J.H. Huber Corp. | 4.72 |
| Barden's clay, a kaolinite | 58.78 |
| Selogen HR | 3.00 |
| Mixture of TMN-6, Triton X-100, and HiSil | 3.00 |

EXAMPLES C-F

1%-10% Granules

A series of four granular formulations of the compound of Example 1 was prepared. In each instance, the appropriate amount of the compound was dissolved in acetone, at the rate of 1 gram of compound per 2 grams of acetone, and the resulting solution was sprayed onto clay under agitation. Thereafter, the acetone was evaporated to provide the final formulation. The clay employed in this series of granular formulations was 30/60 mesh Florex RVM (RVM = regular volatile material), an attapulgite clay produced by the Floridin Co. The compound of Example 1 was estimated to be of 97% purity. The specific granular formulations so produced, each in 50-gram quantity, were as follows.

1% Granule

| Ingredient | Percent by weight |
|---|---|
| Compound of Example 1 | 1.03 |
| clay | 98.97 |

2.5% Granule

| Ingredient | Percent by weight |
|---|---|
| Compound of Example 1 | 2.58 |
| clay | 97.42 |

5.0% Granule

| Ingredient | Percent by weight |
|---|---|
| Compound of Example 1 | 5.15 |
| clay | 94.85 |

10% Granule

| Ingredient | Percent by weight |
|---|---|
| Compound of Example 1 | 10.31 |
| clay | 89.69 |

EXAMPLE G

20% Granule

A 20% granular formulation of the compound of Example 1 was also prepared. The preparation was the same as described in the immediately preceding series, except that the compound was dissolved in acetone, 1.5 grams of acetone per gram of compound of Example 1. Propylene glycol was then added to the

solution, and the acetone was removed by evaporation as in prior examples. The compound of Example 1 which was used in this formulation was estimated to be of 98% purity. The final formulation, in the amount of 105 grams, was as follows.

| Ingredient | Percent by weight |
|---|---|
| Compound of Example 1 | 20.460 |
| propylene glycol | 5.0 |
| clay | 74.54 |

EXAMPLE H

2% Granule

A 2% granular formulation of the compound of Example 1 was also prepared. Again, the procedure used was as reported above. However, 2.5 grams of acetone were employed in dissolving each gram of the compound of Example 1. The compound of Example 1 employed in this formulation was estimated to be of 95% purity. The resulting formulation, in the amount of 19.1 kg, was as follows.

| Ingredient | Percent by weight |
|---|---|
| Compound of Example 1 | 2.1 |
| Propylene glycol | 5.0 |
| clay | 92.9 |

EXAMPLE I

2% Granule

Another 2% granular formulation of the compound of Example 1 was prepared, in the same procedure except that 4 grams of acetone were used in dissolving each gram of the compound of Example 1; the estimated purity of the compound of Example 1 was 97%; and the clay employed was 24/48 mesh Florex, also produced by the Floridin Co.

| Ingredient | Percent by weight |
|---|---|
| Compound of Example 1 | 2.06 |
| Propylene glycol | 5.0 |
| clay | 92.94 |

EXAMPLE J

10% Granule

A 10% granular formulation of the compound of Example 1 was prepared as for the last-described granule, except that the clay employed in this formulation was Oil-Dri 30/40 mesh Mississippi Grey clay RVM (RVM = regular volatile material). The compound of Example 1 which was employed in this formulation was estimated to be of 95% purity. The final formulation, in the amount of 4.5 kg, was as follows.

| Ingredient | Percent by weight |
|---|---|
| Compound of Example 1 | 10.3 |
| Propylene glycol | 5.0 |
| Oil-Dri Mississippi Grey clay RVM, 30/40 mesh | 84.7 |

EXAMPLE K

10% Granule

A granular formulation of the compound of Example 1 was also prepared employing plaster of paris as the carrier. Compound of Example 1 was ground in a mortar and pestle with plaster of paris. Water was then added to make a paste, which was spread thinly over a flat surface, allowed to harden, and broken up into small pieces and sieved, 24/48 "Tyler" mesh. The final formulation was as follows.

10% Granule

| Ingredient | Percent by weight |
|---|---|
| Compound of Example 1 | 8.6 |
| Plaster of paris | 77.3 |
| Theoretical moisture retained if fully dried | 14.1 |

EXAMPLE L

10% Granule

Another granular formulation was prepared in accordance with the foregoing procedures, except that the active ingredient was the compound of Example 77, in estimated purity of 100%. The final formulation was as follows.

| Ingredient | Percent by weight |
|---|---|
| Compound of Example 77 | 10.0 |
| Propylene glycol | 5.0 |
| Oil-Dri Mississippi Grey Clay, 30/40 mesh (RVM) | 85.0 |

EXAMPLE M

10% Granule on various carriers

A series of granular formulations of the compound of Example 85 was prepared. In each instance, the compound, estimated to be of 100% purity, was dissolved in dichloromethane, yielding a 20% solution of the compound of Example 85. A portion of this solution was poured onto each of numerous carriers, mixed well, and air dried to remove the dichloromethane. All of the resulting formulations had the same composition, as follows.

10% Granule

| Ingredient | Percent by weight |
|---|---|
| Compound of Example 85 | 10% |
| carrier, 30/40 mesh | 90% |

The carriers utilized in these formulations, and their sources, were as follows:
Oil-Dri Mississippi Grey clay (this same clay is also referred to as Agsorb RVM-MS);
Oil-Dri Mississippi Brown clay (this same clay is also referred to as Agsorb LVM-MS);
Oil-Dri Georgia white clay - Oil-Dri Corporation
Florex RVM;
Florex LVM - Floridin Company
(LVM = low volatile material)
Agsorb LVM - Oil-Dry Corporation
Attapulgus RVM - Engelhardt Minerals
Bentonite Granular - American Colloid
Pike's Peak clay (9-J) - General Reduction Corp.
KWK Volclay - American Colloid
Lowe's Oran, Missouri clay;
Lowe's Bloomfield, Missouri clay - Lowe's Industrial Products, Inc.
Based on this study, Oil-Dri Mississippi Grey Clay and Oil-Dri Mississippi Brown Clay are regarded as the preferred carriers for the parent compounds. For salts, Florex LVM clay and Lowe's Oran, Missouri clay are preferred.

In view of the fact that the compounds of the present invention exhibit some herbicidal activity, it may be useful to formulate the compounds for insecticidal use in slow-release forms. For example, in using the compounds for the control of corn rootworm, maximum protection against the rootworm is not needed upon germination of the corn seed, but rather several weeks later. Delaying the release of the compound will prevent damage to the germinating corn seedling, while still providing effective control of the corn rootworm at the time it becomes a threat to the corn seedling.

EXAMPLE N

10% Slow Release Granule

A representative slow-release formulation was prepared by dissolving the compound of Example 1, estimated purity of 97%, in acetone at the rate of 1 gram of compound per 4 grams of acetone. The resulting solution was applied to granules of Oil-Dri Mississippi Grey clay, 30/40 mesh, and the acetone was then evaporated off. The granules were then treated with a solution of 5 parts of Carboset 525 (a polyacrylic acid sold by B.F. Goodrich) in 225 parts of a dilute solution of ammonium hydroxide. The treatment consisted of pouring a sixth of the Carboset solution over the granules, mixing well, and drying in a fluid bed drier; the procedure was repeated until all of the Carboset solution had been applied to the granules. The final formulation was as follows.

| Ingredient | Percent by weight |
|---|---|
| Compound of Example 1 | 10.3 |
| Carboset 525 | 5.0 |
| Oil-Dri Mississippi Grey Clay, 30/40 mesh | 84.7 |

EXAMPLE O

10% Slow Release Granule

A 10% slow release granule was made employing the compound of Example 1, Carboset 525, and Florex 20/30 mesh RVM clay. A solution of NaOH was added to another portion of the Carboset 525/$NH_4OH$ solution described in the preceding example, and mixed well. Thereafter, compound of Example 1 was dissolved in acetone and added to the Carboset 525/NaOH solution. The resulting solution was again mixed

well. The solution was then added portionwise to the carrier, by adding one-third of the solution, mixing well and drying, then repeating these steps with the remaining portions. The resulting granule was screened through 20 and 30 mesh screens, and had the following composition.

| Ingredient | Percent by Weight |
|---|---|
| Compound of Example 1 (97% purity) | 10.310 |
| Carboset 525 | 5.000 |
| NaOH | 0.0005 |
| Florex 20/30 RVM Clay | 84.6895 |

EXAMPLE P

2% Slow Release Granule

Four slow release formulations were prepared employing cellulose acetate. The compound of Example 1 was dissolved in acetone and the cellulose acetate was added. The solution was poured over the carrier, mixed well, and air dried. The resulting formulations were as follows.

| Ingredient | Percent by weight |
|---|---|
| Compound of Example 1 | 2.00 |
| Cellulose Acetate | 5.00 |
| Carrier | 93.00 |

The carriers employed in these formulations were the following
Agsorb 30/60 LVM
Attapulgus 30/60 RVM
Florex 30/60 RVM
Oil-Dri Mississippi Grey Clay, 30/60 RVM

EXAMPLE Q

Emulsifiable Concentrate

A 1 lb/gallon emulsifiable concentrate was prepared by dissolving the compound of Example 144, estimated to be of 99% purity, in Panasol AN3N and adding Toximul D.

| Ingredient | Percent by weight |
|---|---|
| Compound of Example 144 | 12.6 |
| Panasol AN3N (a petroleum aromatic solvent supplied by Amoco Chemicals Corp.) | 81.4 |
| Toximul D | 6.0 |
| | 100.0 |

EXAMPLE R

Another 1 lb/gallon emulsifiable concentrate was prepared by dissolving the compound of Example 1 in Panasol AN3N and adding other ingredients. The final formulation was as follows:

| Ingredient | Percent by weight |
|---|---|
| Compound of Example 1 | 12.6 |
| Panasol AN3N | 65.9 |
| Dowanol PM (a glycol monoether sold by Dow Chemical Co.) | 16.5 |
| Toximul H | 3.0 |
| Toximul D | 2.0 |
| | 100.00 |

EXAMPLE S

Yet another 20% wettable powder was prepared with the following composition.

| Ingredient | Percent by weight |
| --- | --- |
| Compound of Example 1, estimated to be of 95% purity | 21.05 |
| Stepanol ME (sodium lauryl sulfonate sold by Stepan Co.) | 5.00 |
| Polyfon O (sugar-free sodium-based sulfonates of Kraft lignin sold by Westvaco Chemicals) | 5.00 |
| Zeolex 7 (an ultra fine hydrous sodium silicoaluminate sold by J.M. Huber Corp.) | 5.00 |
| Barden Clay (an hydrous aluminum silicate sold by J.M. Huber Corp.) | 63.95 |
| | 100.00 |

Starting Materials

As taught above, the compounds of the present invention are prepared by the reaction of an acyl halide and an aniline, 1-aminonaphthalene, or 2-amino-5-nitropyridine:

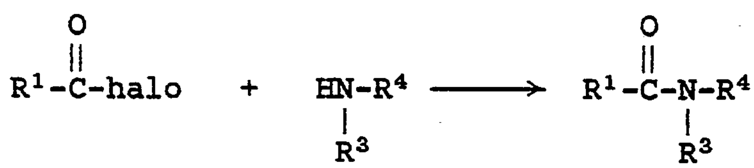

These starting materials are either known compounds or are prepared in known procedures. Essentially all of the anilines are known compounds. 1-Aminonaphthalene and 2-amino-5-nitropyridine are likewise known compounds. Among the acyl halide starting materials, those wherein $R^1$ is perfluorocyclopropyl, perfluorocyclobutyl, perfluorocyclopentyl, and perfluorocyclohexyl with no substituents, are known. The remaining acyl halides are prepared in known procedures. Typically, a substituted benzoyl halide or benzoic acid or a naphthoic acid halide or acid is electrochemically fluorinated to the desired starting material. Because this process results in fluorination of all replaceable groups, a substituent on a benzoyl halide or benzoic acid will be converted to a fluorinated substitutent. Conversion of such substituent groups in the process of electrochemical fluorination is known, as follows:

| Substituent on present starting material | Precursor on benzoic acid or halide | Reference |
|---|---|---|
| $CF_3$ | $CH_3$ | Hudlicky (following table) |
| $OR_f$ | OR | A |
| 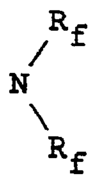 | $NR_2$ | B,C,D |

A. U.S. 2,594,272 (1952)
B. U.K. 666,733 (1952)
C. <u>Chem. Abs.</u>, <u>65</u>, 2140g (1966)
D. <u>Chem. Abs.</u>, <u>62</u>, 16089d (1965)

A general reference on electrochemical fluorination is "Chemistry of Organic Fluorine Compounds" by M. Hudlicky (Horwood Ltd., 1976), especially page 73.

However, as an exception to the foregoing, those starting acyl fluorides which bear a chlorine substitutent require a chlorine-substituted benzoic acid or acid halide, inasmuch as chlorine can survive replacement in the course of electrochemical fluorination. As another exception, it is entirely possible to conduct electrochemical fluorination on a fluorinated or partially fluorinated benzoyl halide. Although the same perfluorinated cyclohexane acyl fluoride is obtained as when beginning with a <u>non</u>-fluorinated benzoyl halide, product yields have sometimes been higher. For example, the acyl fluoride starting material to be employed in Example 140, 4-(trifluoromethoxy)-1,2,2,3,3,4,5,5,6,6-decafluorocyclohexanoyl fluoride, is preferably prepared by electrochemical fluorination of a 4-(trifluoromethoxy)benzoyl halide. Finally, electrochemical fluorination can also be carried out on any of the cycloalkanecarboxylic acids and acid halides, yielding the corresponding perfluorinated cycloalkane acid fluoride.

Electrochemical fluorination of benzoyl halides may result in some rearrangement to isomers which are $CF_3$-substituted, cyclopentane acyl fluorides. Because anilides made from these fluorides share the activity of the present invention, there is no need to remove the rearranged isomers. Also note Example 110, above.

**Claims for the contracting states: BE, CH, DE, FR, GB, IT, LI, LU, NL, SE**

1. A compound of the formula (I)

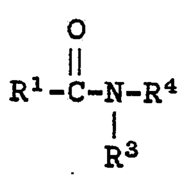

(I)

wherein $R^1$ represents
(1) perfluorocyclopropyl,
(2) perfluorocyclobutyl,
(3) perfluorocycloheptyl,
(4) perfluorodecahydronaphthyl,
(5) fluorocyclopentyl of the formula

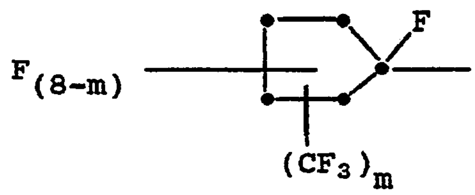

wherein m represents 0, 1, or 2, or
(6) fluorocyclohexyl of the formula

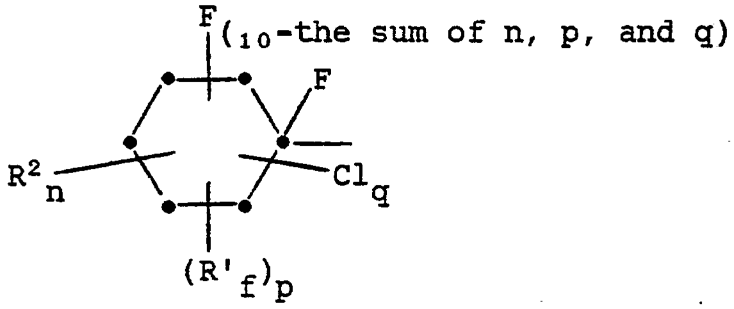

$R^2$ represents $-OR_f$ or

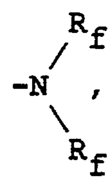

each $R_f$ independently represents perfluorinated loweralkyl of $C_1$-$C_3$, each $R'_f$ independently represents perfluorinated loweralkyl of $C_1$-$C_4$, n represents 0 or 1, each of p and q independently represents 0, 1, or 2, and the sum of n, p, and q is 0-2, inclusive;
$R^3$ represents hydrogen or methyl; and
$R^4$ represents phenyl substituted with
(1) a p-nitro group, or
(2) two to five independently selected $R^5$ groups, where $R^5$ is bromo, chloro, or fluoro, or
(3) two independently selected $R^6$ groups, where $R^6$ is iodo, nitro, cyano, trifluoromethyl, fluorosulfonyl, methylsulfonyl, ethylsulfonyl, carbomethoxy, or carboethoxy, or
(4) two groups, including one $R^5$ group and one $R^6$ group, or
(5) two groups, including one methyl group and one $R^5$, nitro, or fluorosulfonyl group, or
(6) three groups, including two independently selected $R^5$ groups and one $R^6$ group, or
(7) three groups, including two independently selected $R^6$ groups and one $R^5$ group, or
(8) three groups, including two nitro groups and one trifluoromethyl group, or
(9) three groups, including two nitro groups at the 2- and 4-positions and a $C_1$-$C_4$ alkoxy or $C_1$-$C_4$ alkylthio group at the 3- or 5-position, or
(10) three groups, including one methyl group and two groups independently selected from $R^5$, nitro, and fluorosulfonyl, or
(11) two groups, including one nitro $R^5$, or iodo group, and one thiocyanato group, or
$R^4$ represents naphthyl substituted with two or three independently selected $R^5$ or $R^6$ groups, or
$R^4$ represents 5-nitro-2-pyridyl;
and the sodium, potassium, and ammonium salts thereof, wherein ammonium is of the following formula

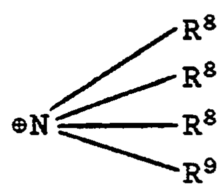

wherein each $R^8$ independently represents alkyl of $C_1$-$C_{20}$, benzyl, 2-hydroxyethyl, 2-hydroxypropyl, or 3-hydroxypropyl, and $R^9$ represents hydrogen or $R^8$, the total number of carbon atoms in all $R^8$ and $R^9$ groups being from 12 to 60, except that when one or more $R^8$ represents 2-hydroxyethyl, 2-hydroxypropyl, or 3-hydroxypropyl, the total number of carbon atoms in all $R^8$ and $R^9$ groups is from 6 to 60.

2. A compound of Claim 1 wherein $R^1$ is as defined in claim 1, provided $R^1$ is not perfluorocycloheptyl or perfluorodecahydronaphthyl, and if p is other than o, $R'_f$ is $CF_3$; and $R^4$ is as defined in claim 1, provided $R^4$ is not phenyl substituted with the groups specified in paragraph (9) or (11) of claim 1.

3. A compound of Claim 2 wherein $R^1$ represents fluorocyclohexyl as defined in paragraph (6).

4. A compound of Claim 3 wherein $R^4$ represents substituted aryl as defined.

5. A compound of Claim 2 which is 2'-bromo-4'-nitro-1,2,2,3,3,4,4,5,5,6,6-undecafluorocyclohexanecarboxanilide or a salt thereof.

6. A compound of Claim 2 which is an ammonium salt, as defined, of 2'-bromo-4'-nitro-1,2,2,3,3,4,4,5,5,6,6-undecafluorocyclohexanecarboxan lide.

7. The compound of Claim 2 which is 2'-bromo-4'-nitro-1,2,2,3,3,4,4,5,5,6,6-undecafluorocyclohexanecarboxanilide, dimethylbis($C_{10}$-$C_{18}$)ammonium salt.

8. A method of inactivating an insect or arachnid which comprises applying to a locus of the insect or arachnid an effective amount of an active agent which is a compound of any one of claims 1 to 7.

9. A method for inhibiting the growth of a plant which comprises applying to the plant an effective amount of a compound of Claim 1.

10. A nematocidal method which comprises applying to nematode-infested soil an effective nematocidal amount of an active agent which is 2'-bromo-4'-nitro-1,2,2, 3,3,4,4,5,5,6,6-undecafluorocyclohexanecarboxanilide or a sodium, potassium, or ammonium salt thereof, wherein ammonium is of the following formula

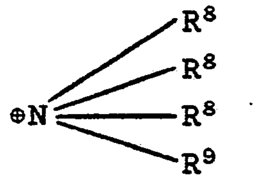

wherein each $R^8$ independently represents alkyl of $C_1$-$C_{20}$, benzyl, 2-hydroxyethyl, 2-hydroxypropyl, or 3-hydroxypropyl, and $R^9$ represents hydrogen or $R^8$, the total number of carbon atoms in all $R^8$ and $R^9$ groups being from 12 to 60, except that when one or more $R^8$ represents 2-hydroxyethyl, 2-hydroxypropyl, or 3-hydroxypropyl, the total number of carbon atoms in all $R^8$ and $R^9$ groups is from 6 to 60.

11. Method of inhibiting a plant pathogenic fungal organism which comprises applying to a locus of the organism an inhibiting amount of an active agent which is a compound of one of the formulae

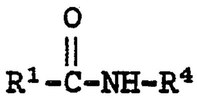

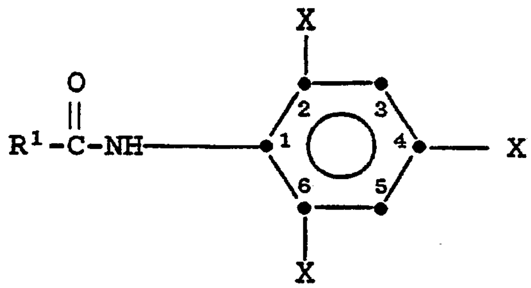

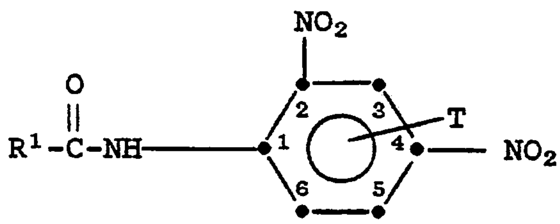

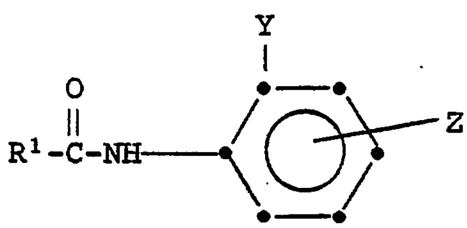

or a salt thereof;
wherein $R^1$ represents
(1) perfluorocyclopropyl,
(2) perfluorocyclobutyl,
(3) perfluorocycloheptyl,
(4) perfluorodecahydronaphthyl,
(5) fluorocyclopentyl of the formula

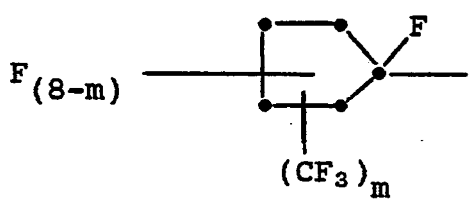

wherein m represents 0, 1, or 2, or
(6) fluorocyclohexyl of the formula

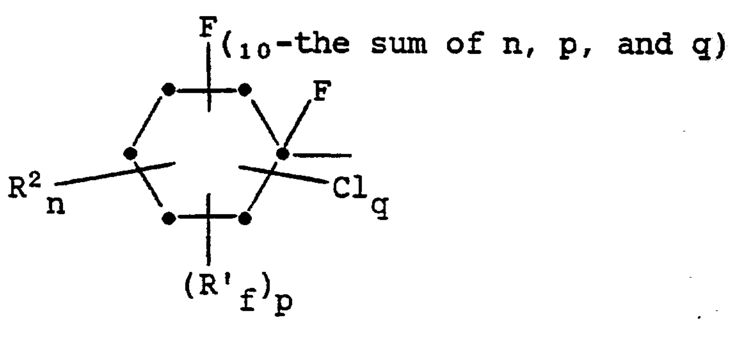

wherein $R^2$ represents $-OR_f$ or

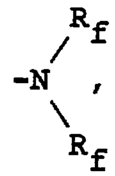

each $R_f$ independently represents perfluorinated loweralkyl of $C_1$-$C_3$, each $R'_f$ independently represents perfluorinated loweralkyl of $C_1$-$C_4$, n represents 0 or 1, each of p and q independently represents 0, 1, or 2, and the sum of n, p, and q is 0-2, inclusive;
$R^4$ is thiocyanatophenyl bearing a single additional substituent which is fluoro, chloro, bromo, iodo or nitro;
each X represents halo, independently selected except that no more than one X can represent iodo; T represents fluoro, $C_1$-$C_4$ alkoxy, or $C_1$-$C_4$ alkylthio, located at either the 3- or 5-position; Y represents halo, nitro, or methyl; Z represents nitro or fluorosulfonyl; and the salt is a sodium, potassium or ammonium salt, wherein ammonium is of the following formula

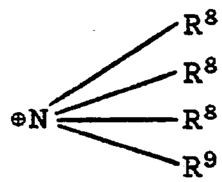

wherein each $R^8$ independently represents alkyl of $C_1$-$C_{20}$, benzyl, 2-hydroxyethyl, 2-hydroxypropyl, or 3-hydroxypropyl, and $R^9$ represents hydrogen or $R^8$, the total number of carbon atoms in all $R^8$ and $R^9$ groups being from 12 to 60, except that when one or more $R^8$ represents 2-hydroxyethyl, 2-hydroxypropyl, or 3-hydroxypropyl, the total number of carbon atoms in all $R^8$ and $R^9$ groups is from 6 to 60.

12. The method of Claim 11 wherein the active agent is 2',4',6'-trichloro-1,2,2,3,3,4,4,5,5, 6,6-undecafluorocyclohexanecarboxanilide or a salt thereof.

13. The method of Claim 11 wherein the active agent is 2',4'-dinitro-5'-fluoro-1,2,2,3,3,4,4, 5,5,6,6-undecafluorocyclohexanecarboxanilide or a salt thereof.

14. The method of Claim 11 wherein the active agent is 2',5'-dinitro-1,2,2,3,3,4,4,5,5,6,6,-undecafluorocyclohexanecarboxanili e or a salt thereof.

15. The method of claim 11 wherein the active agent is 2'-nitro-4'-thiocyanato-1,2,2,3,3,4,4,5,5,6,6-undecafluorocyclohexaneca boxanilide or a salt thereof.

16. An insecticidal or arachnicidal formulation comprising, as active ingredient, an effective amount of a compound of any one of claims 1 to 7 in combination with an agriculturally acceptable adjuvant.

17. A nematocidal formulation comprising an active agent as described in claim 10 in combination with an agriculturally acceptable adjuvant.

18. A herbicidal formulation comprising, as active ingredient, a compound of any one of claims 1 to 7 in combination with an agriculturally acceptable adjuvant.

19. An antifungal formulation comprising an active agent as described in any one of claims 11 to 14 in combination with an agriculturally acceptable adjuvant.

20. A process for preparing a compound of formula (I) or a salt thereof as defined in claim 1, which comprises
reacting an acyl halide of the formula

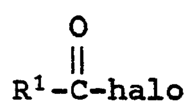

with an aniline, 1-aminonaphthalene, or 2-amino-5-nitropyridine of the formula

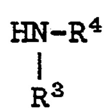

in a nonreactive organic solvent, and,

if necessary, methylating the resulting compound of formula I to provide a compound of formula I wherein $R^3$ is methyl, and,
if necessary, salifying the resulting compound.

**Claims for the contracting state: AT**

1. An agrichemical formulation comprising, as active ingredient, a compound of the formula (I)

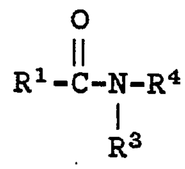

(I)

wherein $R^1$ represents
(1) perfluorocyclopropyl,
(2) perfluorocyclobutyl,
(3) perfluorocycloheptyl,
(4) perfluorodecahydronaphthyl,
(5) fluorocyclopentyl of the formula

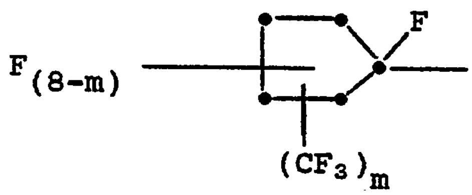

wherein m represents 0, 1, or 2, or
(6) fluorocyclohexyl of the formula

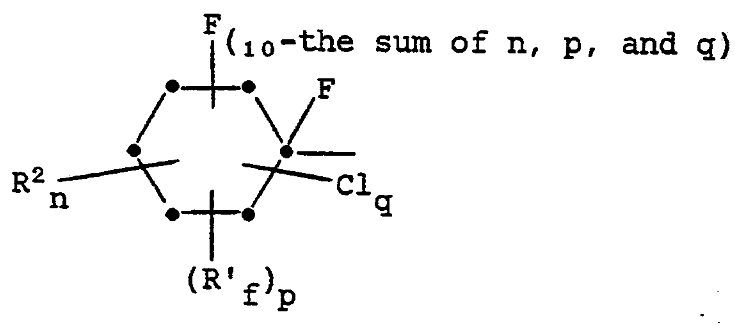

$R^2$ represents $-OR_f$ or

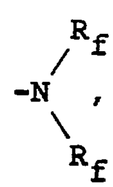

each $R_f$
independently represents perfluorinated loweralkyl of $C_1$-$C_3$, each $R'_f$ independently represents perfluorinated loweralkyl of $C_1$-$C_4$, n represents 0 or 1, each of p and q independently represents 0, 1, or 2, and the sum of n, p, and q is 0-2, inclusive;
$R^3$ represents hydrogen or methyl; and
$R^4$ represents phenyl substituted with
(1) a p-nitro group, or
(2) two to five independently selected $R^5$ groups, where $R^5$ is bromo, chloro, or fluoro, or
(3) two independently selected $R^6$ groups, where $R^6$ is iodo, nitro, cyano, trifluoromethyl, fluorosulfonyl, methylsulfonyl, ethylsulfonyl, carbomethoxy, or carboethoxy, or
(4) two groups, including one $R^5$ group and one $R^6$ group, or

(5) two groups, including one methyl group and one $R^5$, nitro, or fluorosulfonyl group, or
(6) three groups, including two independently selected $R^5$ groups and one $R^6$ group, or
(7) three groups, including two independently selected $R^6$ groups and one $R^5$ group, or
(8) three groups, including two nitro groups and one trifluoromethyl group, or
(9) three groups, including two nitro groups at the 2- and 4-positions and a $C_1$-$C_4$ alkoxy or $C_1$-$C_4$ alkylthio group at the 3- or 5-position, or
(10) three groups, including one methyl group and two groups independently selected from $R^5$, nitro, and fluorosulfonyl, or
(11) two groups, including one nitro $R^5$, or iodo group, and one thiocyanato group, or
$R^4$ represents naphthyl substituted with two or three independently selected $R^5$ or $R^6$ groups, or $R^4$ represents 5-nitro-2-pyridyl;
and the sodium, potassium, and ammonium salts thereof, wherein ammonium is of the following formula

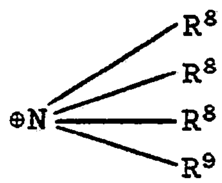

wherein each $R^8$ independently represents alkyl of $C_1$-$C_{20}$, benzyl, 2-hydroxyethyl, 2-hydroxypropyl, or 3-hydroxypropyl, and $R^9$ represents hydrogen or $R^8$, the total number of carbon atoms in all $R^8$ and $R^9$ groups being from 12 to 60, except that when one or more $R^8$ represents 2-hydroxyethyl, 2-hydroxypropyl, or 3-hydroxypropyl, the total number of carbon atoms in all $R^8$ and $R^9$ groups is from 6 to 60, in combination with an agriculturally acceptable adjuvant.

2. A formulation of claim 1 wherein the active ingredient is of formula (I) wherein $R^1$ is as defined in claim 1, provided $R^1$ is not perfluorocycloheptyl or perfluorodecahydronaphthyl, and if p is other than o, $R'_f$ is $CF_3$; and $R^4$ is as defined in claim 1, provided $R^4$ is not phenyl substituted with the groups specified in paragraph (9) or (11) of claim 1.

3. A formulation of Claim 2 wherein $R^1$ represents fluorocyclohexyl as defined in paragraph (6).

4. A formulation of Claim 3 wherein $R^4$ represents substituted aryl as defined.

5. A formulation of Claim 2 wherein the active ingredient is 2'-bromo-4'-nitro-1,2,2,3,3,4,45,5,6,6,-undecafluorocyclohexanecarboxan lide or a salt thereof.

6. A formulation of Claim 2 wherein the active ingredient is an ammonium salt, as defined, of 2'-bromo-4'-nitro-1,2,2,3,3,4,4,5,5,6,6-undecafluorocyclohexanecarboxan lide.

7. The formulation of Claim 2 wherein the active ingredient is 2'-bromo-4'-nitro-1,2,2,3,3,4,4,5,5,6,6-undecafluorocyclohexanecarboxan lide, dimethylbis($C_{10}$-$C_{18}$)ammonium salt.

8. A method of inactivating an insect or arachnid which comprises applying to a locus of the insect or arachnid an effective amount of an active ingredient described in any one of claims 1 to 7.

9. A method for inhibiting the growth of a plant which comprises applying to the plant an effective amount of active ingredient described in claim 1.

10. A nematocidal method which comprises applying to nematode-infested soil an effective nematocidal amount of an active agent which is 2'-bromo-4'-nitro-1,2,2, 3,3,4,4,5,5,6,6-undecafluorocyclohexanecarboxanilide or a sodium, potassium, or ammonium salt thereof, wherein ammonium is of the following formula

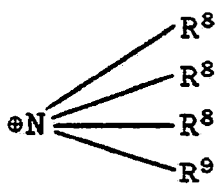

wherein each $R^8$ independently represents alkyl of $C_1$-$C_{20}$, benzyl, 2-hydroxyethyl, 2-hydroxypropyl, or 3-hydroxypropyl, and $R^9$ represents hydrogen or $R^8$, the total number of carbon atoms in all $R^8$ and $R^9$ groups being from 12 to 60, except that when one or more $R^8$ represents 2-hydroxyethyl, 2-hydroxypropyl, or 3-hydroxypropyl, the total number of carbon atoms in all $R^8$ and $R^9$ groups is from 6 to 60.

11. Method of inhibiting a plant pathogenic fungal organism which comprises applying to a locus of the organism an inhibiting amount of an active agent which is a compound of one of the formulae

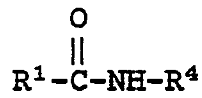

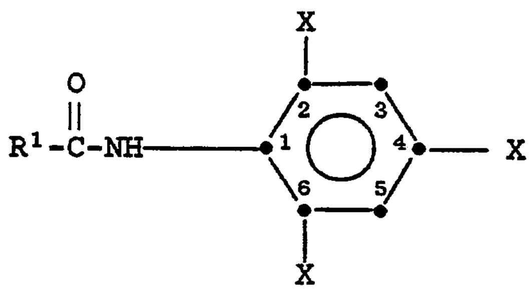

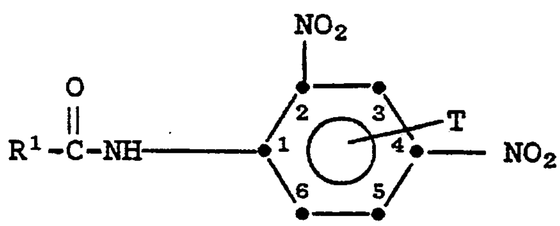

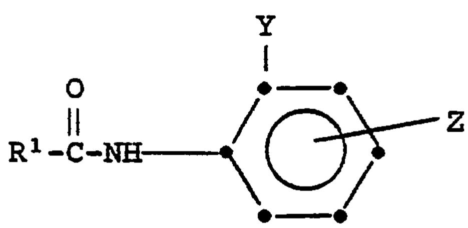

or a salt thereof;
wherein $R^1$ represents
(1) perfluorocyclopropyl,
(2) perfluorocyclobutyl,
(3) perfluorocycloheptyl,
(4) perfluorodecahydronaphthyl,
(5) fluorocyclopentyl of the formula

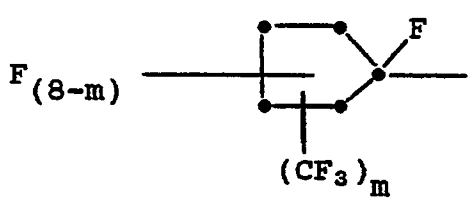

wherein m represents 0, 1, or 2, or
(6) fluorocyclohexyl of the formula

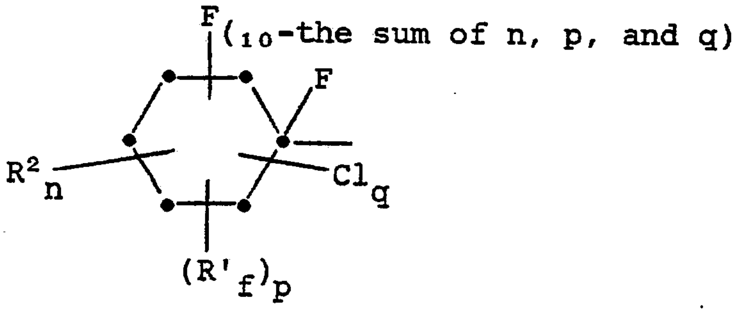

wherein $R^2$ represents $-OR_f$ or

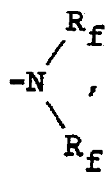

each $R_f$
independently represents perfluorinated loweralkyl of $C_1$-$C_3$, each $R'_f$ independently represents perfluorinated loweralkyl of $C_1$-$C_4$, n represents 0 or 1, each of p and q independently represents 0, 1, or 2, and the sum of n, p, and q is 0-2, inclusive;
$R^4$ is thiocyanatophenyl bearing a single additional substituent which is fluoro, chloro, bromo, iodo, or nitro;
each X represents halo, independently selected except that no more than one X can represent iodo; T represents fluoro, $C_1$-$C_4$ alkoxy, or $C_1$-$C_4$ alkylthio, located at either the 3- or 5-position; Y represents halo, nitro, or methyl; Z represents nitro or fluorosulfonyl; and the salt is a sodium, potassium or ammonium salt, wherein ammonium is of the following formula

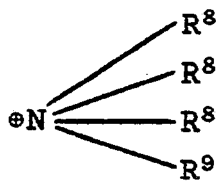

wherein each $R^8$ independently represents alkyl of $C_1$-$C_{20}$, benzyl, 2-hydroxyethyl, 2-hydroxypropyl, or 3-hydroxypropyl, and $R^9$ represents hydrogen or $R^8$, the total number of carbon atoms in all $R^8$ and $R^9$ groups being from 12 to 60, except that when one or more $R^8$ represents 2-hydroxyethyl, 2-hydroxypropyl, or 3-hydroxypropyl, the total number of carbon atoms in all $R^8$ and $R^9$ groups is from 6 to 60.

12. The method of Claim 11 wherein the active agent is 2',4',6'-trichloro-1,2,2,3,3,4,4,5,5, 6,6-undecafluorocyclohexanecarboxanilide or a salt thereof.

13. The method of Claim 11 wherein the active agent is 2',4'-dinitro-5'-fluoro-1,2,2,3,3,4,4, 5,5,6,6-undecafluorocyclohexanecarboxanilide or a salt thereof.

14. The method of Claim 11 wherein the active agent is 2',5'-dinitro-1,2,2,3,3,4,4,5,5,6,6-undecafluorocyclohexanecarboxanilid or a salt thereof.

15. An insecticidal or arachnicidal formulation comprising, the active ingredient described in any one of claims 1 to 7 in combination with an agriculturally acceptable adjuvant.

16. A nematocidal formulation comprising an active agent as described in claim 10 in combination with an agriculturally acceptable adjuvant.

17. A herbicidal formulation comprising, an active agent described in any one of claims 1 to 7 in combination with an agriculturally acceptable adjuvant.

18. An antifungal formulation comprising an active agent as described in any one of claims 11 to 14 in combination with an agriculturally acceptable adjuvant.

19. A process for preparing a compound of formula (I) or a salt thereof as defined in claim 1, which comprises
reacting an acyl halide of the formula

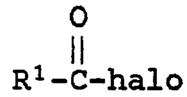

with an aniline, 1-aminonaphthalene, or 2-amino-5-nitropyridine of the formula

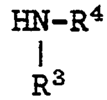

in a nonreactive organic solvent, and,
if necessary, methylating the resulting compound of formula I to provide a compound of formula I wherein $R^3$ is methyl, and,
if necessary, salifying the resulting compound.

**Patentansprüche für die Vertragsstaaten: BE, CH, DE, FR, GB, IT, LI, LU, NL, SE**

1. Verbindung der Formel (I)

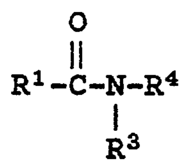

worin $R^1$ folgendes bedeutet:
(1) Perfluorcyclopropyl,
(2) Perfluorcyclobutyl,
(3) Perfluorcycloheptyl,
(4) Perfluordecahydronaphthyl,
(5) Fluorcyclopentyl der Formel

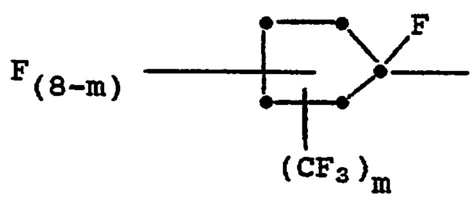

worin m 0, 1 oder 2 bedeutet, oder
(6) Fluorcyclohexyl der Formel

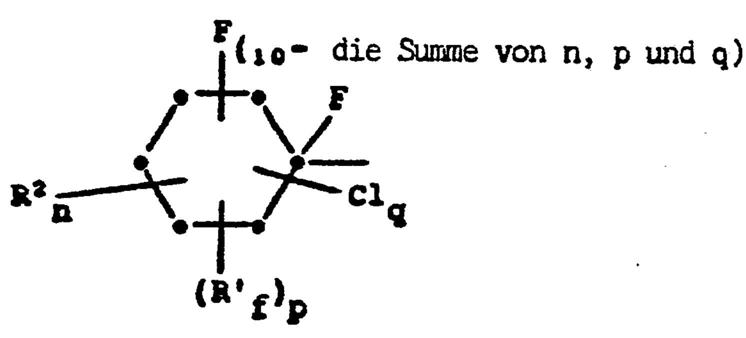

worin $R^2$ die Bedeutung $-OR_f$ oder

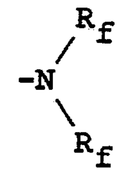

hat, wobei die Reste $R_f$ jeweils unabhängig voneinander perfluoriertes nieder-Alkyl mit $C_1$–$C_3$ bedeuten, wobei die einzelnen Reste $R'_f$ unabhängig voneinander perfluoriertes nieder-Alkyl mit $C_1$–$C_4$ bedeuten, n den Wert 0 oder 1 hat, die einzelnen Reste p und q unabhängig voneinander den Wert 0, 1 oder 2 haben und die Summe von n, p und q 0 bis einschließlich 2 ist;

$R^3$ Wasserstoff oder Methyl bedeutet; und

$R^4$ Phenyl bedeutet, das substituiert ist durch

(1) eine p-Nitrogruppe oder

(2) zwei bis fünf unabhängig voneinander ausgewählte $R^5$-Gruppen, wobei $R^5$ Brom, Chlor oder Fluor bedeutet, oder

(3) zwei unabhängig voneinander ausgewählte $R^6$-Gruppen, wobei $R^6$ Jod, Nitro, Cyano, Trifluormethyl, Fluorsulfonyl, Methylsulfonyl, Ethylsulfonyl, Carbomethoxy oder Carboethoxy bedeutet, oder

(4) zwei Gruppen unter Einschluß einer $R^5$-Gruppe und einer $R^6$-Gruppe, oder

(5) zwei Gruppen, unter Einschluß einer Methylgruppe und einer $R^5$-, Nitro- oder Fluorsulfonylgruppe, oder

(6) drei Gruppen, unter Einschluß von zwei unabhängig voneinander ausgewählten $R^5$-Gruppen und einer $R^6$-Gruppe, oder

(7) drei Gruppen, unter Einschluß von zwei unabhängig voneinander ausgewählten $R^6$-Gruppen und einer $R^5$-Gruppe, oder

(8) drei Gruppen, unter Einschluß von zwei Nitrogruppen und einer Trifluormethylgruppe, oder

(9) drei Gruppen, unter Einschluß von zwei Nitrogruppen in der 2- und 4-Stellung und einer $C_1$–$C_4$-Alkoxy- oder $C_1$–$C_4$-Alkylthiogruppe in der 3- oder 5-Stellung, oder

(10) drei Gruppen, unter Einschluß einer Methylgruppe und zwei unabhängig voneinander unter $R^5$, Nitro und Fluorsulfonyl ausgewählten Gruppen, oder

(11) zwei Gruppen, unter Einschluß einer Thiocyanatogruppe und einer Nitro-, $R^5$- oder Jodgruppe, oder

$R^4$ Naphthyl bedeutet, das mit zwei oder drei unabhängig voneinander ausgewählten $R^5$ oder $R^6$-Gruppen substituiert ist, oder

$R^4$ 5-Nitro-2-pyridyl bedeutet;

und die Natrium-, Kalium- und Ammoniumsalze davon, wobei es sich bei Ammonium um einen Rest der folgenden Formel handelt

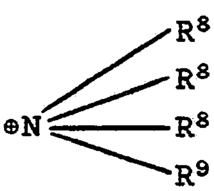

wobei die einzelnen Reste $R^8$ jeweils unabhängig voneinander Alkyl mit $C_1$–$C_{20}$, Benzyl, 2-Hydroxyethyl, 2-Hydroxypropyl oder 3-Hydroxypropyl bedeuten, und $R^9$ Wasserstoff oder $R^8$ bedeutet, wobei die Gesamtzahl der Kohlenstoffatome in sämtlichen $R^8$- und $R^9$-Gruppen 12 bis 60 beträgt, mit der Ausnahme, daß, wenn einer oder mehrere der Reste $R^8$ 2-Hydroxyethyl, 2-Hydroxypropyl oder 3-Hydroxypropyl bedeuten, die Gesamtzahl der Kohlenstoffatome in sämtlichen $R^8$- und $R^9$-Gruppen 6 bis 60 beträgt.

2. Verbindung nach Anspruch 1, wobei $R^1$ die in Anspruch 1 definierte Bedeutung hat, mit der Maßgabe, daß $R^1$ nicht Perfluorcycloheptyl oder Perfluordecahydronaphthyl bedeutet und, wenn p einen anderen Wert als 0 hat, $R'_f$ $CF_3$ bedeutet; und $R^4$ die in Anspruch 1 definierte Bedeutung hat, mit der Maßgabe, daß $R^4$ nicht mit den in Abschnitt (9) oder (11) von Anspruch 1 angegebenen Gruppen substituiertes Phenyl bedeutet.

3. Verbindung nach Anspruch 2, wobei $R^1$ Fluorcyclohexyl gemäß der Definition in Abschnitt (6) bedeutet.

4. Verbindung nach Anspruch 3, wobei $R^4$ substituiertes Aryl gemäß der angegebenen Definition bedeutet.

5. Verbindung nach Anspruch 2, bei der es sich um 2'-Brom-4'-nitro-1,2,2,3,3,4,4,5,5,6,6-undecafluorcyclohexancarboxanilid oder ein Salz davon handelt.

6. Verbindung nach Anspruch 2, bei der es sich um ein Ammoniumsalz der angegebenen Definition von 2'-Brom-4'-nitro-1,2,2,3,3,4,4,5,5,6,6-undecafluorcyclohexancarboxanilid handelt.

7. Verbindung nach Anspruch 2, bei der es sich um 2'-Brom-4'-nitro-1,2,2,3,3,4,4,5,5,6,6-undecafluorcyclohexancarboxanilid-dimethyl-bis-($C_{10}$–$C_{18}$)-ammoniumsalz handelt.

8. Verfahren zum Inaktivieren eines Insekts oder spinnenartigen Tiers, das das Aufbringen einer wirksamen Menge eines Wirkstoffs, bei der es sich um eine Verbindung nach einem der Ansprüche 1 bis 7 handelt, auf eine Stelle, an der sich das Insekt oder das spinnenartige Tier befindet, umfaßt.

9. Verfahren zur Hemmung des Wachstums einer Pflanze, das das Aufbringen einer wirksamen Menge einer Verbindung nach Anspruch 1 auf die Pflanze umfaßt.

10. Nematozides Verfahren, das das Aufbringen einer wirksamen, nematoziden Menge eines Wirkstoffs, bei dem es sich um 2'-Brom-4'-nitro-1,2,2,3,3,4,4,5,5,6,6-undecafluorcyclohexancarboxanilid oder ein Natrium-, Kalium- oder Ammoniumsalz handelt, auf Boden mit Nematodenbefall umfaßt, wobei die Ammoniumgruppe die folgende Formel aufweist

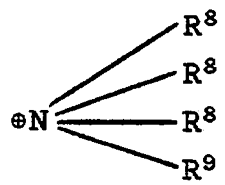

wobei die Reste $R^8$ jeweils unabhängig voneinander Alkyl mit $C_1$–$C_{20}$, Benzyl, 2-Hydroxyethyl, 2-Hydroxypropyl oder 3-Hydroxypropyl bedeuten und $R^9$ Wasserstoff oder $R^8$ bedeutet, wobei die Gesamtzahl der Kohlenstoffatome in sämtlichen $R^8$- und $R^9$-Gruppen 12 bis 60 beträgt, mit der Ausnahme, daß, wenn einer oder mehrere der Reste $R^8$ 2-Hydroxyethyl, 2-Hydroxypropyl oder 3-Hydroxypropyl bedeuten, die Gesamtzahl der Kohlenstoffatome in sämtlichen $R^8$- und $R^9$-Gruppen 6 bis 60 beträgt.

11. Verfahren zur Hemmung eines pflanzenpathogenen Pilzorganismus, das das Aufbringen einer hemmenden Menge eines Wirkstoffs auf eine Stelle, wo sich der Organismus befindet, umfaßt, wobei es sich um eine Verbindung der folgenden Formeln

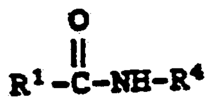

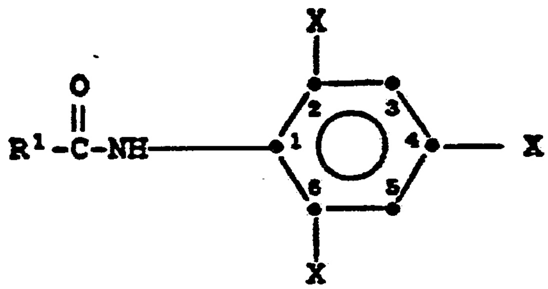

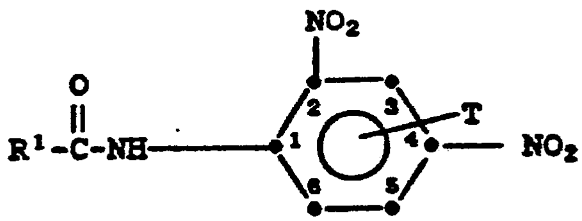

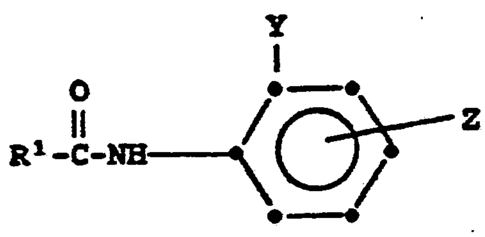

oder ein Salz davon handelt,
worin R[1] folgendes bedeutet:

(1) Perfluorcyclopropyl,
(2) Perfluorcyclobutyl,
(3) Perfluorcycloheptyl,
(4) Perfluordecahydronaphthyl,
(5) Fluorcyclopentyl der Formel

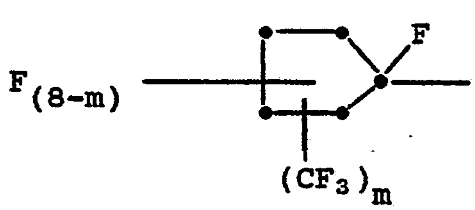

worin m 0, 1 oder 2 bedeutet, oder
(6) Fluorcyclohexyl der Formel

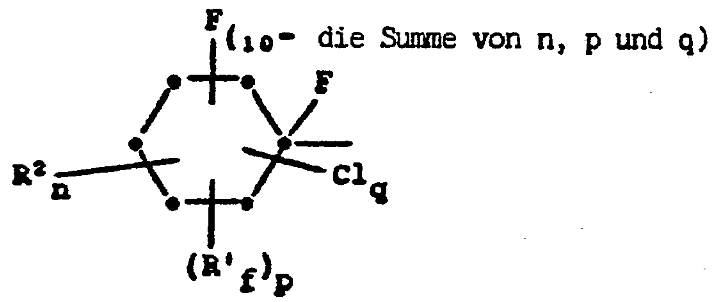

worin $R^2$ die Bedeutung $-OR_f$ oder

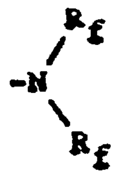

hat,
wobei die Reste $R_f$ jeweils unabhängig voneinander perfluoriertes nieder-Alkyl mit $C_1$–$C_3$ bedeuten, wobei die einzelnen Reste $R'_f$ unabhängig voneinander perfluoriertes nieder-Alkyl mit $C_1$–$C_4$ bedeuten, n den Wert 0 oder 1 hat, die einzelnen Reste p und q unabhängig voneinander den Wert 0, 1 oder 2 haben und die Summe von n, p und q 0 bis einschließlich 2 ist;
$R^4$ Thiocyanatophenyl mit einem einzigen weiteren Substituenten, bei dem es sich um Fluor, Chlor, Brom, Jod oder Nitro handelt, bedeutet;
wobei die einzelnen Reste X jeweils Halogen bedeuten, die unabhängig voneinander ausgewählt sind, mit der Ausnahme, daß nicht mehr als einer der Reste X Jod bedeuten kann; T Fluor, $C_1$–$C_4$-Alkoxy oder $C_1$–$C_4$-Alkylthio an der 3- oder 5-Stellung bedeutet; Y Halogen, Nitro oder Methyl bedeutet; Z Nitro oder Fluorsulfonyl bedeutet; und wobei es sich beim Salz um ein Natrium-, Kalium- oder Ammoniumsalz handelt, wobei das Ammonium die folgende Formel aufweist

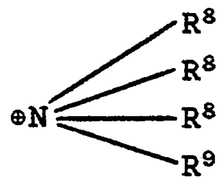

wobei die Reste $R^8$ jeweils unabhängig voneinander Alkyl mit $C_1$–$C_{20}$, Benzyl, 2-Hydroxyethyl, 2-Hydroxypropyl oder 3-Hydroxypropyl bedeuten und $R^9$ Wasserstoff oder $R^8$ bedeutet, wobei die Gesamtzahl der Kohlenstoffatome in sämtlichen $R^8$- und $R^9$-Gruppen 12 bis 60 beträgt, mit der Ausnahme, daß, wenn einer oder mehrere der Reste $R^8$ 2-Hydroxyethyl, 2-Hydroxypropyl oder 3-Hydroxypropyl bedeuten, die Gesamtzahl der Kohlenstoffatome in sämtlichen $R^8$- und $R^9$-Gruppen 6 bis 60 beträgt.

12. Verfahren nach Anspruch 11, wobei es sich beim Wirkstoff um 2′,4′,6′-Trichlor-1,2,2,3,3,4,4,5,5,6,6-undecafluorcyclohexancarbonxanilid oder ein Salz davon handelt.

13. Verfahren nach Anspruch 11, wobei es sich beim Wirkstoff um 2′,4′-Dinitro-5′-fluor-1,2,2,3,3,4,4,5,5,6,6-undecafluorcyclohexancarboxanilid oder ein Salz davon handelt.

14. Verfahren nach Anspruch 11, wobei es sich beim Wirkstoff um 2′,5′-Dinitro-1,2,2,3,3,4,4,5,5,6,6-undecafluorcyclohexancarboxanilid oder ein Salz davon handelt.

15. Verfahren nach Anspruch 11, wobei es sich beim Wirkstoff um 2′-Nitro-4′-thiocyanato-1,2,2,3,3,4,4,5,5,6,6-undecafluorcyclohexancarboxanilid oder ein Salz davon handelt.

16. Insektizide oder arachnizide Zubereitung, enthaltend als Wirkstoff eine wirksame Menge einer Verbindung nach einem der Ansprüche 1 bis 7 in Kombination mit einem landwirtschaftlich verträglichen Adjuvans.

17. Nematozide Zubereitung, enthaltend einen Wirkstoff nach Anspruch 10 in Kombination mit einem landwirtschaftlich verträglichen Adjuvans.

18. Herbizide Zubereitung, enthaltend als Wirkstoff eine Verbindung nach einem der Ansprüche 1 bis 7 in Kombination mit einem landwirtschaftlich verträglichen Adjuvans.

19. Antifungale Zubereitung, enthaltend einen Wirkstoff nach einem der Ansprüche 11 bis 14 in Kombination mit einem landwirtschaftlich verträglichen Adjuvans.

20. Verfahren zur Herstellung einer Verbindung der Formel (I) oder eines Salzes davon gemäß der Definition in Anspruch 1, das folgendes umfaßt:
Umsetzen eines Acylhalogenids der Formel

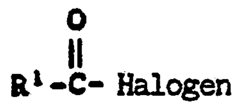

mit einem Anilin, 1-Aminonaphthalin oder 2-Amino-5-nitropyridin der Formel

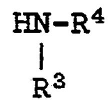

in einem nicht-reaktiven, organischen Lösungsmittel und
gegebenenfalls Methylieren der erhaltenen Verbindung der Formel I unter Bereitstellung einer Verbindung der Formel I, in der $R^3$ Methyl bedeutet und
gegebenenfalls Überführen der erhaltenen Verbindung in ein Salz.

**Patentansprüche für den Vertragsstaat: AT**

1. Agrochemische Zubereitung, enthaltend als Wirkstoff eine Verbindung der Formel (I)

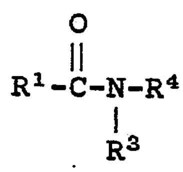

(I)

worin $R^1$ folgendes bedeutet:
(1) Perfluorcyclopropyl,
(2) Perfluorcyclobutyl,
(3) Perfluorcycloheptyl,
(4) Perfluordecahydronaphthyl,
(5) Fluorcyclopentyl der Formel

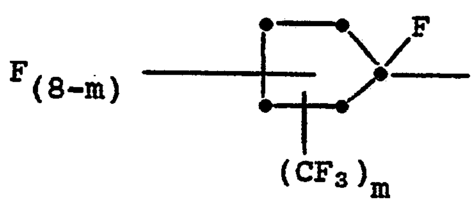

worin m 0, 1 oder 2 bedeutet, oder
(6) Fluorcyclohexyl der Formel

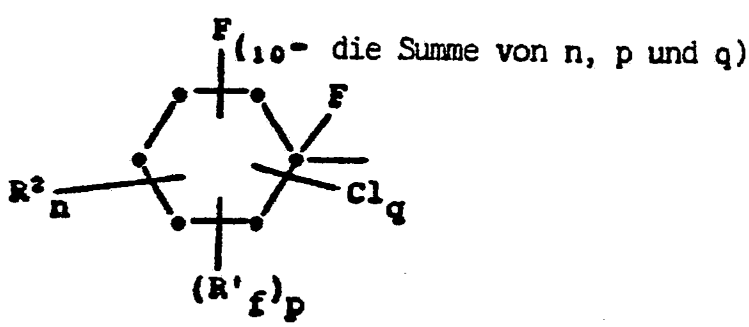

worin $R^2$ die Bedeutung $-OR_f$ oder

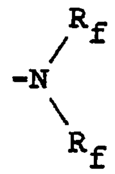

hat,
wobei die Reste $R_f$ jeweils unabhängig voneinander perfluoriertes nieder-Alkyl mit $C_1$–$C_3$ bedeuten, wobei die einzelnen Reste $R'_f$ unabhängig voneinander perfluoriertes nieder-Alkyl mit $C_1$–$C_4$ bedeuten, n den Wert 0 oder 1 hat, die einzelnen Reste p und q unabhängig voneinander den Wert 0, 1 oder 2 haben und die Summe von n, p und q 0 bis einschließlich 2 ist;
$R^3$ Wasserstoff oder Methyl bedeutet; und
$R^4$ Phenyl bedeutet, das substituiert ist durch
(1) eine p-Nitrogruppe oder
(2) zwei bis fünf unabhängig voneinander ausgewählte $R^5$-Gruppen, wobei $R^5$ Brom, Chlor oder Fluor bedeutet, oder
(3) zwei unabhängig voneinander ausgewählte $R^6$-Gruppen, wobei $R^6$ Jod, Nitro, Cyano, Trifluormethyl, Fluorsulfonyl, Methylsulfonyl, Ethylsulfonyl, Carbomethoxy oder Carboethoxy bedeutet, oder
(4) zwei Gruppen unter Einschluß einer $R^5$-Gruppe und einer $R^6$-Gruppe, oder
(5) zwei Gruppen, unter Einschluß einer Methylgruppe und einer $R^5$-, Nitro- oder Fluorsulfonylgruppe, oder
(6) drei Gruppen, unter Einschluß von zwei unabhängig voneinander ausgewählten $R^5$-Gruppen und einer $R^6$-Gruppe, oder
(7) drei Gruppen, unter Einschluß von zwei unabhängig voneinander ausgewählten $R^6$-Gruppen und einer $R^5$-Gruppe, oder
(8) drei Gruppen, unter Einschluß von zwei Nitrogruppen und einer Trifluormethylgruppe, oder
(9) drei Gruppen, unter Einschluß von zwei Nitrogruppen in der 2- und 4-Stellung und einer $C_1$–$C_4$-Alkoxy- oder $C_1$–$C_4$-Alkylthiogruppe in der 3- oder 5-Stellung, oder
(10) drei Gruppen, unter Einschluß einer Methylgruppe und zwei unabhängig voneinander unter $R^5$, Nitro und Fluorsulfonyl ausgewählten Gruppen, oder
(11) zwei Gruppen, unter Einschluß einer Nitro-, $R^5$- oder Jodgruppe und einer Thiocyanatogruppe oder
$R^4$ Naphthyl bedeutet, das mit zwei oder drei unabhängig voneinander ausgewählten $R^5$ oder $R^6$-Gruppen substituiert ist, oder
$R^4$ 5-Nitro-2-pyridyl bedeutet;
und die Natrium-, Kalium- und Ammoniumsalze davon, wobei es sich bei Ammonium um einen Rest der folgenden Formel handelt

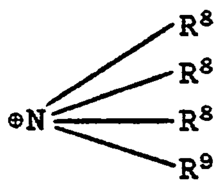

wobei die einzelnen Reste $R^8$ jeweils unabhängig voneinander Alkyl mit $C_1$–$C_{20}$, Benzyl, 2-Hydroxyethyl, 2-Hydroxypropyl oder 3-Hydroxypropyl bedeuten, und $R^9$ Wasserstoff oder $R^8$ bedeutet, wobei die Gesamtzahl der Kohlenstoffatome in sämtlichen $R^8$- und $R^9$-Gruppen 12 bis 60 beträgt, mit der Aushahme, daß, wenn einer oder mehrere der Reste $R^8$ 2-Hydroxyethyl, 2-Hydroxypropyl oder 3-Hydroxypropyl bedeuten, die Gesamtzahl der Kohlenstoffatome in sämtlichen $R^8$- und $R^9$-Gruppen 6 bis 60 beträgt.

2. Zubereitung nach Anspruch 1, wobei der Wirkstoff die Formel (I) aufweist, worin $R^1$ die in Anspruch 1 definierte Bedeutung hat, mit der Maßgabe, daß $R^1$ nicht Perfluorcycloheptyl oder Perfluordecahydronaphthyl bedeutet und, wenn p einen anderen Wert als 0 hat, $R'_f$ $CF_3$ bedeutet; und $R^4$ die in Anspruch 1 definierte Bedeutung hat, mit der Maßgabe, daß $R^4$ nicht mit den in Abschnitt (9) oder (11) von Anspruch 1 angegebenen Gruppen substituiertes Phenyl bedeutet.

3. Zubereitung nach Anspruch 2, wobei $R^1$ Fluorcyclohexyl gemäß der Definition in Abschnitt (6) bedeutet.

4. Zubereitung nach Anspruch 3, wobei $R^4$ substituiertes Aryl gemäß der angegebenen Definition bedeutet.

5. Zubereitung nach Anspruch 2, wobei es sich beim Wirkstoff um 2'-Brom-4'-nitro-1,2,2,3,3,4,4,5,5,6,6-undecafluorcyclohexancarboxanilid oder ein Salz davon handelt.

6. Zubereitung nach Anspruch 2, wobei es sich beim Wirkstoff um ein Ammoniumsalz der angegebenen Definition von 2'-Brom-4'-nitro-1,2,2,3,3,4,4,5,5,6,6-undecafluorcyclohexancarboxanilid handelt.

7. Zubereitung nach Anspruch 2, wobei es sich beim Wirkstoff um 2'-Brom-4'-nitro-1,2,2,3,3,4,4,5,5,6,6-undecafluorcyclohexancarboxanilid-dimethyl-bis-($C_{10}$–$C_{18}$)-ammoniumsalz handelt.

8. Verfahren zum Inaktivieren eines Insekts oder spinnenartigen Tiers, das das Aufbringen einer wirksamen Menge eines Wirkstoffs, bei der es sich um eine Verbindung nach einem der Ansprüche 1 bis 7 handelt, auf eine Stelle, an der sich das Insekt oder das spinnenartige Tier befindet, umfaßt.

9. Verfahren zur Hemmung des Wachstums einer Pflanze, das das Aufbringen einer wirksamen Menge einer Verbindung nach Anspruch 1 auf die Pflanze umfaßt.

10. Nematozides Verfahren, das das Aufbringen einer wirksamen, nematoziden Menge eines Wirkstoffs, bei dem es sich um 2'-Brom-4'-nitro-1,2,2,3,3,4,4,5,5,6,6-undecafluorcyclohexancarboxanilid oder ein Natrium-, Kalium- oder Ammoniumsalz handelt, auf Boden mit Nematodenbefall umfaßt, wobei die Ammoniumgruppe die folgende Formel aufweist

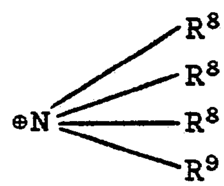

wobei die Reste $R^8$ jeweils unabhängig voneinander Alkyl mit $C_1$–$C_{20}$, Benzyl, 2-Hydroxyethyl, 2-Hydroxypropyl oder 3-Hydroxypropyl bedeuten und $R^9$ Wasserstoff oder $R^8$ bedeutet, wobei die Gesamtzahl der Kohlenstoffatome in sämtlichen $R^8$- und $R^9$-Gruppen 12 bis 60 beträgt, mit der Ausnahme, daß, wenn einer oder mehrere der Reste $R^8$ 2-Hydroxyethyl, 2-Hydroxypropyl oder 3-Hydroxypropyl bedeuten, die Gesamtzahl der Kohlenstoffatome in sämtlichen $R^8$- und $R^9$-Gruppen 6 bis 60 beträgt.

11. Verfahren zur Hemmung eines pflanzenpathogenen Pilzorganismus, das das Aufbringen einer hemmenden Menge eines Wirkstoffs auf eine Stelle, wo sich der Organismus befindet, umfaßt, wobei es sich um eine Verbindung der folgenden Formeln

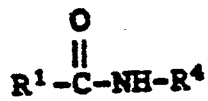

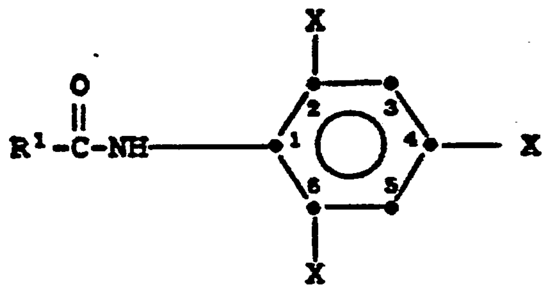

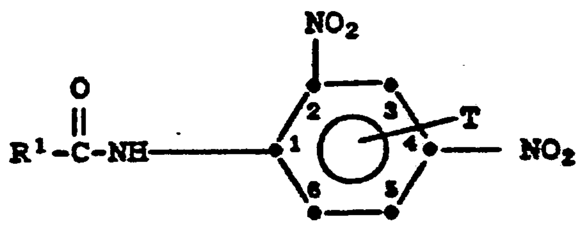

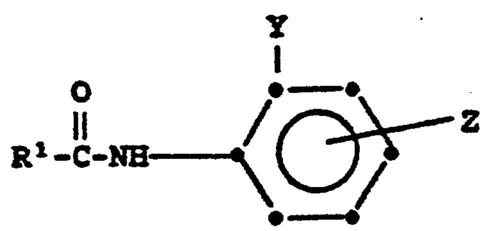

oder ein Salz davon handelt,
worin $R^1$ folgendes bedeutet:

(1) Perfluorcyclopropyl,
(2) Perfluorcyclobutyl,
(3) Perfluorcycloheptyl,
(4) Perfluordecahydronaphthyl,
(5) Fluorcyclopentyl der Formel

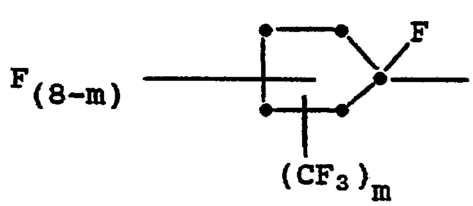

worin m 0, 1 oder 2 bedeutet, oder
(6) Fluorcyclohexyl der Formel

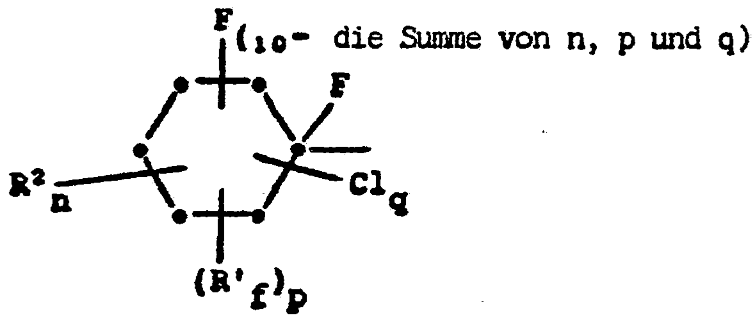

worin $R^2$ die Bedeutung $-OR_f$ oder

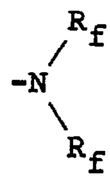

hat,

wobei die Reste $R_f$ unabhängig voneinander perfluoriertes nieder-Alkyl mit $C_1$–$C_3$ bedeuten, wobei die einzelnen Reste $R'_f$ unabhängig voneinander perfluoriertes nieder-Alkyl mit $C_1$–$C_4$ bedeuten, n den Wert 0 oder 1 hat, die einzelnen Reste p und q unabhängig voneinander den Wert 0, 1 oder 2 haben und die Summe von n, p und q 0 bis einschließlich 2 ist;

$R^4$ Thiocyanatophenyl mit einem einzigen weiteren Substituenten, bei dem es sich um Fluor, Chlor, Brom, Jod oder Nitro handelt, bedeutet;

wobei die einzelnen Reste X jeweils Halogen bedeuten, die unabhängig voneinander ausgewählt sind, mit der Ausnahme, daß nicht mehr als einer der Reste X Jod bedeuten kann; T Fluor, $C_1$–$C_4$-Alkoxy oder $C_1$–$C_4$-Alkylthio an der 3- oder 5-Stellung bedeutet; Y Halogen, Nitro oder Methyl bedeutet; Z Nitro oder Fluorsulfonyl bedeutet; und wobei es sich beim Salz um ein Natrium-, Kalium- oder Ammoniumsalz handelt, wobei das Ammonium die folgende Formel aufweist

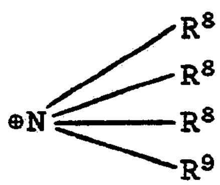

wobei die Reste $R^8$ jeweils unabhängig voneinander Alkyl mit $C_1$–$C_{20}$, Benzyl, 2-Hydroxyethyl, 2-Hydroxypropyl oder 3-Hydroxypropyl bedeuten und $R^9$ Wasserstoff oder $R^8$ bedeutet, wobei die Gesamtzahl der Kohlenstoffatome in sämtlichen $R^8$- und $R^9$-Gruppen 12 bis 60 beträgt, mit der Ausnahme, daß, wenn einer oder mehrere der Reste $R^8$ 2-Hydroxyethyl, 2-Hydroxypropyl oder 3-Hydroxypropyl bedeuten, die Gesamtzahl der Kohlenstoffatome in sämtlichen $R^8$- und $R^9$-Gruppen 6 bis 60 beträgt.

12. Verfahren nach Anspruch 11, wobei es sich beim Wirkstoff um 2′,4′,6′-Trichlor-1,2,2,3,3,4,4,5,5,6,6-undecafluorcyclohexancarbonxanilid oder ein Salz davon handelt.

13. Verfahren nach Anspruch 11, wobei es sich beim Wirkstoff um 2′,4′-Dinitro-5′-fluor-1,2,2,3,3,4,4,5,5,6,6-undecafluorcyclohexancarboxanilid oder ein Salz davon handelt.

14. Verfahren nach Anspruch 11, wobei es sich beim Wirkstoff um 2′,5′-Dinitro-1,2,2,3,3,4,4,5,5,6,6-undecafluorcyclohexancarboxanilid oder ein Salz davon handelt.

15. Insektizide oder arachnizide Zubereitung, enthaltend als Wirkstoff eine wirksame Menge einer Verbindung nach einem der Ansprüche 1 bis 7 in Kombination mit einem landwirtschaftlich verträglichen Adjuvans.

16. Nematozide Zubereitung, enthaltend einen Wirkstoff nach Anspruch 10 in Kombination mit einem landwirtschaftlich verträglichen Adjuvans.

17. Herbizide Zubereitung, enthaltend als Wirkstoff eine Verbindung nach einem der Ansprüche 1 bis 7 in Kombination mit einem landwirtschaftlich verträglichen Adjuvans.

18. Antifungale Zubereitung, enthaltend einen Wirkstoff nach einem der Ansprüche 11 bis 14 in Kombination mit einem landwirtschaftlich verträglichen Adjuvans.

19. Verfahren zur Herstellung einer Verbindung der Formel (I) oder eines Salzes davon gemäß der Definition in Anspruch 1, das folgendes umfaßt:
Umsetzen eines Acylhalogenids der Formel

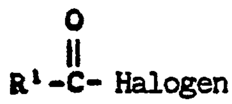

mit einem Anilin, 1-Aminonaphthalin oder 2-Amino-5-nitropyridin der Formel

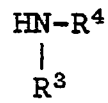

in einem nicht-reaktiven, organischen Lösungsmittel und
gegebenenfalls Methylieren der erhaltenen Verbindung der Formel I unter Bereitstellung einer Verbindung der Formel I, in der $R^3$ Methyl bedeutet und
gegebenenfalls Überführen der erhaltenen Verbindung in ein Salz.

**Revendications pour les états contractants: BE, CH, DE, FR, GB, IT, LI, LU, NL, SE**

1. Composé de la formule (I)

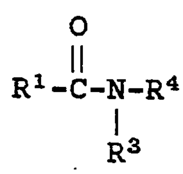

(I)

dans laquelle $R^1$ représente
(1) un radical perfluorocyclopropyle
(2) un radical perfluorocyclobutyle
(3) un radical perfluorocycloheptyle
(4) un radical perfluorodécahydronaphtyle
(5) un radical fluorocyclopentyle suivant la formule

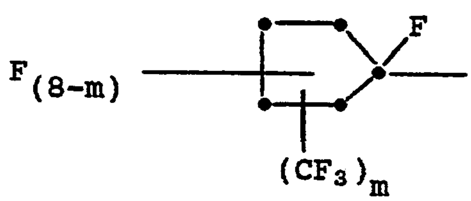

dans laquelle m représente 0, 1 ou 2, ou
(6) un radical fluorocyclohexyle suivant la formule

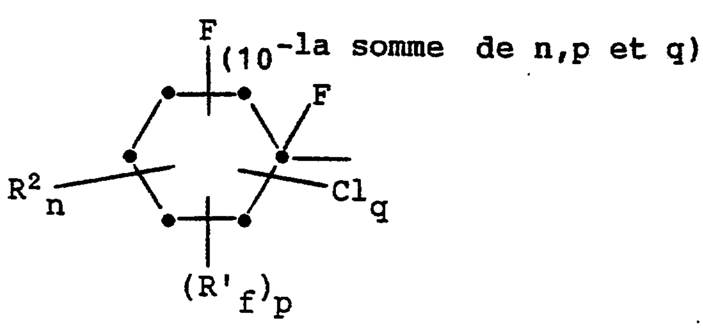

dans laquelle
$R^2$ représente $-OR_f$ ou

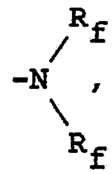

chaque $R_f$ représentant, indépendamment l'un de l'autre, un radical alkyle inférieur perfluoré en $C_1$–$C_3$, chaque $R'_f$ représentant, indépendamment l'un de l'autre, un radical alkyle inférieur perfluoré en $C_1$–$C_4$, n représentant 0 ou 1, chaque p et q représentant, indépendamment l'un de l'autre, 0, 1 ou 2 et la somme de n, p et q étant comprise entre 0 et 2:

$R^3$ représente de l'hydrogène ou un radical méthyle; et

$R^4$ représente un radical phényle substitué avec

(1) un groupe p-nitro, ou

(2) deux à cinq groupes $R^5$ choisis indépendamment, dans lesquels $R^5$ est un radical bromo, chloro ou fluoro, ou

(3) deux groupes $R^6$ choisis indépendamment, dans lesquels $R^6$ représente un groupe iodo, nitro, cyano, trifluorométhyle, fluorosulfonyle, méthylsulfonyle, éthylsulfonyle, carbométhoxy ou carboéthoxy, ou

(4) deux groupes, y compris un groupe $R^5$ et un groupe $R^6$, ou

(5) deux groupes, y compris un groupe méthyle et un $R^5$, un groupe nitro ou fluorosulfonyle,

(6) trois groupes, y compris deux groupes $R^5$ choisis indépendamment et un groupe $R^6$,

(7) trois groupes, y compris deux groupes $R^6$ choisis indépendamment et un groupe $R^5$,

(8) trois groupes, y compris deux groupes nitro et un groupe trifluorométhyle, ou

(9) trois groupes, y compris deux groupes nitro en positions 2 et 4 et un groupe alcoxy en $C_1$–$C_4$ ou alkylthio en $C_1$–$C_4$ en positions 3 ou 5, ou

(10) trois groupes, y compris un groupe méthyle et deux groupes choisis indépendamment parmi $R^5$, nuitro et fluorosulfonyle, ou

(11) deux groupes, y compris un groupe thiocyanato et un groupe nitro, $R^5$ ou iodo, ou

$R^4$ représente un radical naphtyle substitué par deux ou trois groupes $R^5$ ou $R^6$ choisis indépendamment,

$R^4$ représente un radical 5-nitro-2-pyridyle; et les sels sodique, potassique et ammonique de celui-ci, dans lequel l'ammonium présente la formule suivante

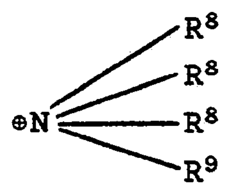

dans laquelle chaque $R^8$ représente indépendamment un radical alkyle en $C_1$–$C_{20}$, un radical benzyle, un radical 2-hydroxyéthyle, un radical 2-hydroxypropyle ou un radical 3-hydroxypropyle et $R^9$ représente de l'hydrogène ou $R^8$, le nombre total d'atomes de carbone dans tous les groupes $R^8$ et $R^9$ étant compris entre 12 et 60 sauf que un ou plusieurs R8 représentent un radical 2-hydroxyéthyle, un radical 2-hydroxypropyle ou un radical 3-hydroxy-propyle, le nombre total d'atomes de carbone dans tous les groupes $R^8$ et $R^9$ étant compris entre 6 et 60.

2. Composé selon la revendication 1, dans lequel $R^1$ est tel que défini à la revendication 1 à condition que $R^1$ ne soit pas un radical perfluorocycloheptyle ou perfluorodécahydronaphtyle et que p soit différent de 0, $R'_f$ ,représente $CF_3$ et $R^4$ est tel que défini à la revendication 1, à condition que $R^4$ ne soit pas un radical phényle substitué par les groupes mentionnés aux § (9) ou (11) de la revendication 1.

3. Composé selon la revendication 2, dans lequel $R^1$ représente un radical fluorocyclohexyle tel que défini au § (6).

4. Composé selon la revendication 3, dans lequel $R^4$ représente un aryle substitué tel que défini.

5. Composé selon la revendication 2, qui est le 2′-bromo-4′-nitro-1,2,3,3,4,4,5,5,6,6-undécafluorocyclohexanecarboxanilide ou un de ses sels.

6. Composé selon la revendication 2, qui est un sel ammonique tel que défini du 2′-bromo-4′-nitro-1,2,3,3,4,4,5,5,6,6-undécafluorocyclohexanecarboxanilide.

7. Comosé selon la revendication 2, qui est le sel de diméthyl-bis($C_{10}$–$C_{18}$)ammonium du 2′-bromo-4′-nitro-1,2,2,3,3,4,4,5,5,6,6-undécafluorocyclohexanecarboxanilide.

8. Procédé d'inactivation d'un insecte ou d'un arachnide qui comprend l'application à un endroit de vie de l'insecte ou de l'arachnide d'une quantité efficace d'un agent actif qui est un composé de l'une des revendications 1 à 7.

9. Procédé pour inhiber la croissance d'une plante, qui comprend l'application à la plante d'une quantité efficace d'un composé de la revendication 1.

10. Procédé nématocide qui consiste à appliquer aux sols infectés par les nématodes une quantité nématocide efficace d'un agent actif qui est le 2'-bromo-4'-nitro-1,2,2,3,3,4,4,5,5,6,6-undécafluorocyclohexanecarboxanilide ou un sel sodique, potassique ou ammonique de celui-ci, dans lequel l'ammonium présente la formule suivante

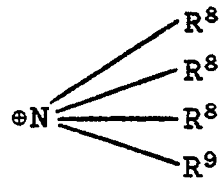

dans laquelle chaque $R^8$ représente indépendamment un radical alkyle en $C_1$–$C_{20}$, un radical benzyle, un radical 2-hydroxyéthyle, un radical 2-hydroxypropyle ou un radical 3-hydroxypropyle et $R^9$ représente de l'hydrogène ou $R^8$, le nombre total d'atomes de carbone dans tous les groupes $R^8$ et $R^9$ étant compris entre 12 et 60 sauf que un ou plusieurs $R^8$ représentent un radical 2-hydroxyéthyle, un radical 2-hydroxypropyle ou un radical 3-hydroxypropyle, le nombre total d'atomes de carbone dans tous les groupes $R^8$ et $R^9$ étant compris entre 6 et 60.

11. Procédé d'inhibition d'un champignon pathogène des plantes, qui consiste à appliquer à un endroit de vie de cet organisme une quantité inhibitrice de l'agent actif qui est un composé suivant l'une des formules suivantes:

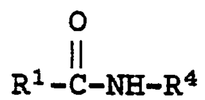

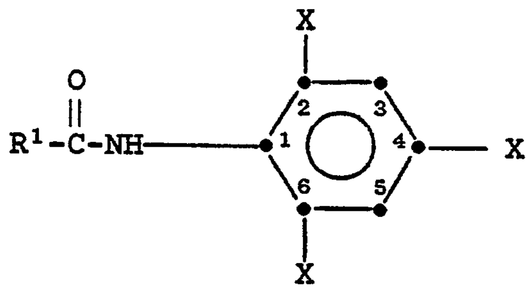

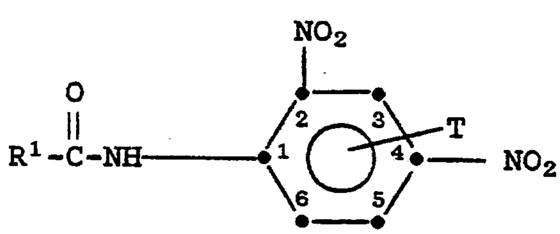

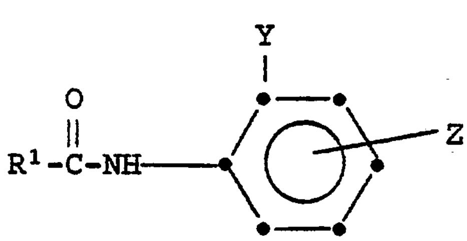

ou un sel de celui-ci;
dans lesquels $R^1$ représente
(1) un radical perfluorocyclopropyle
(2) un radical perfluorocyclobutyle
(3) un radical perfluorocycloheptyle
(4) un radical perfluorodécahydronaphtyle
(5) un radical fluorocyclopentyle suivant la formule

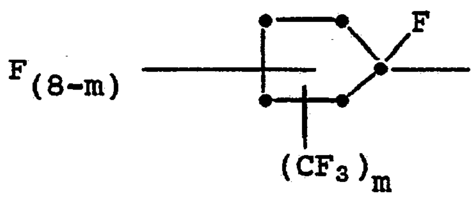

dans laquelle m représente 0, 1 ou 2, ou
(6) un radical fluorocyclohexyle suivant la formule

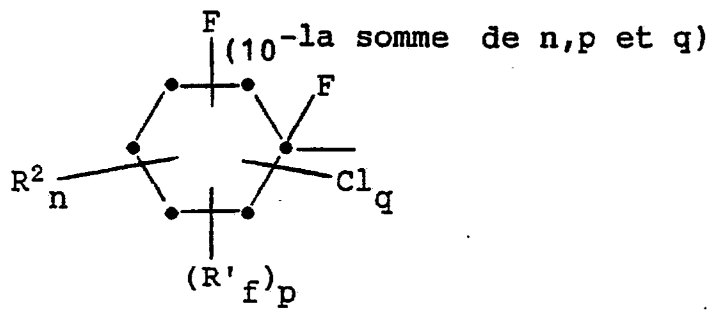

dans laquelle
$R^2$ représente $-OR_f$ ou

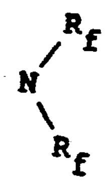

chaque $R_f$ représentant, indépendamment l'un de l'autre, un radical alkyle inférieur perfluoré en $C_1$–$C_3$, chaque $R'_f$ représentant, indépendamment l'un de l'autre, un radical alkyle inférieur perfluoré en $C_1$–$C_4$, n représentant 0 ou 1, chaque p et q représentant, indépendamment l'un de l'autre, 0, 1 ou 2 et la somme de n, p et q étant comprise entre 0 et 2:
$R^4$ est un radical thiocyanatophényle portant un seul substituant supplémentaire qui est un radical fluoro, chloro, bromo, iodo ou nitro, chaque X représente un radical halo, choisi indépendamment, excepté qu'un X au maximum peut représenter un radical iodo, T représente un radical fluoro, un radical alcoxy en $C_1$–$C_4$ ou un radical alkylthio en $C_1$–$C_4$ situé en position 3 ou 5, Y représente un radical halo, nitro ou méthyle, Z représente un radical nitro ou fluorosulfonyle et le sel est un sel sodique, potassique ou ammonique dans lequel l'alluminum présente la formule suivante

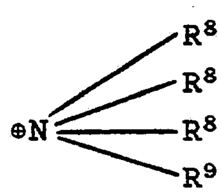

dans laquelle chaque $R^8$ représente indépendamment un radical alkyle en $C_1$–$C_{20}$, un radical benzyle, un radical 2-hydroxyéthyle, un radical 2-hydroxypropyle ou un radical 3-hydroxypropyle et $R^9$ représente de l'hydrogène ou $R^8$, le nombre total d'atomes de carbone dans tous les groupes $R^8$ et $R^9$ étant

compris entre 12 et 60 sauf que un ou plusieurs $R^8$ représentent un radical 2-hydroxyéthyle, un radical 2-hydroxypropyle ou un radical 3-hydroxypropyle, le nombre total d'atomes de carbone dans tous les groupes $R^8$ et $R^9$ étant compris entre 6 et 60.

12. Procédé selon la revendication 11, dans lequel l'agent actif est le 2',4',6'-trichloro-1,2,2,3,3,4,4,5,5,6,6-undécafluorocyclohexanecarboxanilide ou un sel de celui-ci.

13. Procédé selon la revendication 11, dans l'agent actif est le 2',4'-dinitro-5'-fluoro-1,2,2,3,3,4,4,5,5,6,6-undécafluorocyclohexanecarboxanilide ou un sel de celui-ci.

14. Procédé selon la revendication 11, dans lequel l'agent actif est le 2'-5'-dinitro-1,2,2,3,3,4,4,5,5,6,6-undécafluorocyclohexanecarboxanilide ou un sel de celui-ci.

15. Procédé selon la revendication 11, dans lequel l'agent actif est le 2'-nitro-4'-thiocyanato-1,2,2,3,3,4,4,5,5,6,6-undécafluorocyclohexanecarboxanilide ou un sel de celui-ci.

16. Formulation insecticide ou arachnicide comprenant un ingrédient actif, une quantité efficace d'un composé selon l'une quelconque des revendications 1 à 7 en combinaison avec un additif acceptable en agriculture.

17. Formulation nématocide comprenant un agent actif tel que décrit à la revendication 10 en combinaison avec un additif acceptable en agriculture.

18. Formulation herbicide comprenant comme ingrédient actif un composé selon l'une des revendications quelconques 1 à 7, en combinaison avec un additif acceptable en agriculture.

19. Formulation fongicide comprenant un agent actif tel que décrit dans l'une des revendications quelconques 11 à 14, en combinaison avec un additif acceptable en agriculture.

20. Procédé pour préparer un composé de la formule (I) ou un sel de celui-ci, tel que défini à la revendication 1, qui comprend
la réaction d'un halogénure d'acyle suivant la formule

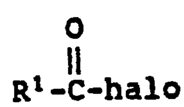

avec une aniline, le 1-aminonaphtalène ou la 2-amino-5-nitropyridine suivant la formule

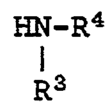

dans un solvant organique non réactif, et
si nécessaire, la méthylation du composé résultant de la formule (I) afin d'obtenir un composé de la formule (I), dans lequel $R^3$ est un radical méthyle et
si nécessaire, la salification du composé résultant.

**Revendications pour l'état contractant: AT**

1. Formulation agrochimique comprenant comme ingrédient actif un composé de la formule (I)

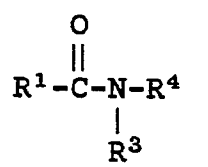 (I)

dans laquelle $R^1$ représente
(1) un radical perfluorocyclopropyle
(2) un radical perfluorocyclobutyle
(3) un radical perfluorocycloheptyle
(4) un radical perfluorodécahydronaphtyle
(5) un radical fluorocyclopentyle suivant la formule

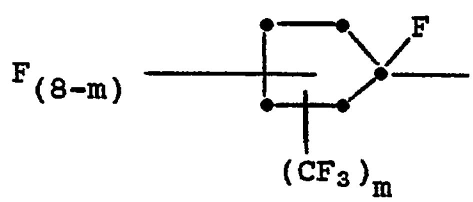

dans laquelle m représente 0, 1 ou 2, ou
(6) un radical fluorocyclohexyle suivant la formule

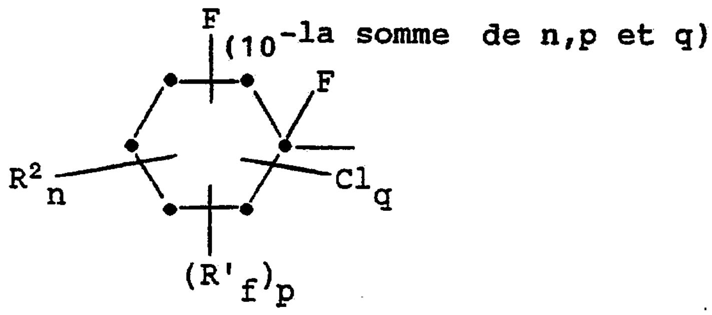

dans laquelle
$R^2$ représente $-OR_f$ ou

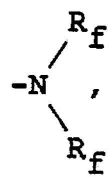

chaque $R_f$ représentant, indépendamment l'un de l'autre, un radical alkyle inférieur perfluoré en $C_1$–$C_3$, chaque $R'_f$ représentant, indépendamment l'un de l'autre, un radical alkyle inférieur perfluoré en $C_1$–$C_4$, n représentant 0 ou 1, chaque p et q représentant, indépendamment l'un de l'autre, 0, 1 ou 2 et la somme de n, p et q étant comprise entre 0 et 2 inclusivement:

$R_3$ représente de l'hydrogène ou un radical méthyle; et

$R^4$ représente un radical phényle substitué
(1) un groupe p-nitro, ou
(2) deux à cinq groupes $R^5$ choisis indépendamment, dans lesquels $R^5$ est un radical bromo, chloro ou fluoro, ou
(3) deux groupes $R^6$ choisis indépendamment, dans lesquels $R^6$ représente un groupe iodo, nitro, cyano, trifluorométhyle, fluorosulfonyle, méthylsulfonyle, éthylsulfonyle, carbométhoxy ou carboéthoxy, ou
(4) deux groupes, y compris un groupe $R^5$ et un groupe $R^6$, ou
(5) deux groupes, y compris un groupe méthyle et un $R^5$, un groupe nitro ou fluorosulfonyle, ou
(6) trois groupes, y compris deux groupes $R^5$ choisis indépendamment et un groupe $R^6$, ou
(7) trois groupes, y compris deux groupes $R^6$ choisis indépendamment et un groupe $R^5$, ou
(8) trois groupes, y compris deux groupes nitro et un groupe trifluorométhyle, ou
(9) trois groupes, y compris deux groupes nitro en positions 2 et 4 et un groupe alcoxy en $C_1$–$C_4$ ou alkylthio en $C_1$–$C_4$ en positions 3 ou 5, ou
(10) trois groupes, y compris un groupe méthyle et deux groupes choisis indépendamment parmi $R^5$, nitro et fluorosulfonyle, ou
(11) deux groupes, y compris un groupe thiocyanato et un groupe nitro, $R^5$ ou iodo, ou
$R^4$ représente un radical naphtyle substitué par deux ou trois groupes $R^5$ ou $R^6$ choisis indépendamment, ou
$R^4$ représente un radical 5-nitro-2-pyridyle; et les sels sodique, potassique et ammonique de celui-ci, dans lequel l'ammonium présente la formule suivante

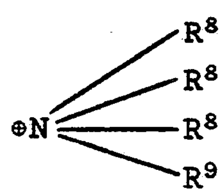

dans laquelle chaque $R^8$ représente indépendamment un radical alkyle en $C_1$–$C_{20}$, un radical benzyle, un radical 2-hydroxyéthyle, un radical 2-hydroxypropyle ou un radical 3-hydroxypropyle et $R^9$ représente de l'hydrogène ou $R^8$, le nombre total d'atomes de carbone dans tous les groupes $R^8$ et $R^9$ étant

compris entre 12 et 60 sauf que si un ou plusieurs $R^8$ représentent un radical 2-hydroxyéthyle, un radical 2-hydroxypropyle ou un radical 3-hydroxypropyle, le nombre total d'atomes de carbone dans tous les groupes $R^8$ et $R^9$ est compris entre 6 et 60, en combinaison avec un adjuvant acceptable en agriculture.

2. Formulation selon la revendication 1, dans laquelle l'ingrédient actif est suivant la formule (I) dans laquelle $R^1$ est tel que défini à la revendication 1 à condition que $R^1$ ne soit pas un radical perfluorocycloheptyle ou perfluorodécahydronaphtyle et que p soit différent de 0, $R'_f$ représente $CF_3$ et $R^4$ est tel que défini à la revendicyation 1, à condition que $R^4$ ne soit pas un radical phényle substitué par les groupes mentionnés aux § (9) ou (11) de la revendication 1.

3. Formulation selon la revendication 2, dans laquelle $R^1$ représente un radical fluorocyclohexyle tel que défini au § (6).

4. Formulation selon la revendication 3, dans laquelle $R^4$ représente un radical aryle substitué tel que défini.

5. Formulation selon la revendication 2, dans laquelle l'ingrédient actif est le 2'-bromo-4'-nitro-1,2,2,3,3,4,4,5,5,6,6-undécafluorocyclohexanecarboxanilide ou un de ses sels.

6. Formulation selon la revendication 2, dans laquelle l'ingrédient actif est un sel ammonique tel que défini du 2'-bromo-4'-nitro-1,2,2,3,3,4,4,5,5,6,6-undécafluorocyclohexanecarboxanilide.

7. Formulation selon la revendication 2, dans laquelle l'ingrédient actif est le sel de diméthyl-bis($C_{10}$–$C_{18}$)ammonium du 2'-bromo-4'-nitro-1,2,2,3,3,4,4,5,5,6,6-undécafluorocyclohexanecarboxanilide.

8. Procédé d'inactivation d'un insecte ou d'un arachnide qui comprend l'application à un habitat de l'insecte ou de l'arachnide d'une quantité efficace d'un ingrédient actif tel que défini dans l'une quelconque des revendications 1 à 7.

9. Procédé pour inhiber la croissance d'une plante, qui comprend l'application à la plante d'une quantité efficace d'un ingrédient actif tel que décrit à la revendication 1.

10. Procédé nématocide qui consiste à appliquer aux sols infestés par les nématodes une quantité nématocide efficace d'un agent actif qui est le 2'-bromo-4'-nitro-1,2,2,3,3,4,4,5,5,6,6-undécafluorocyclohexanecarboxanilide ou un sel sodique, potassique ou ammonique de celui-ci, dans lequel l'ammonium présente la formule suivante

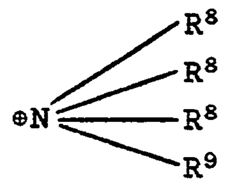

dans laquelle chaque $R^8$ représente indépendamment un radical alkyle en $C_1$–$C_{20}$, un radical benzyle, un radical 2-hydroxyéthyle, un radical 2-hydroxypropyle ou un radical 3-hydroxypropyle et $R^9$ représente de l'hydrogène ou $R^8$, le nombre total d'atomes de carbone dans tous les groupes $R^8$ et $R^9$ étant compris entre 12 et 60 sauf que si un ou plusieurs $R^8$ représentent un radical 2-hydroxyéthyle, un radical 2-hydroxypropyle ou un radical 3-hydroxypropyle, le nombre total d'atomes de carbone dans tous les groupes $R^8$ et $R^9$ est compris entre 6 et 60.

11. Procédé d'inhibition d'un organisme fongique pathogène des plantes, qui consiste à appliquer à un habitat de cet organisme une quantité inhibitrice de l'agent actif qui est un composé suivant l'une des formules suivantes:

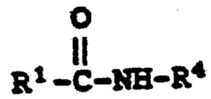

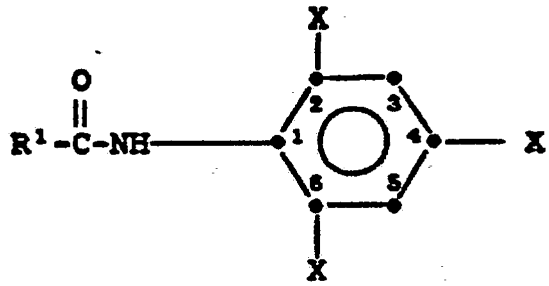

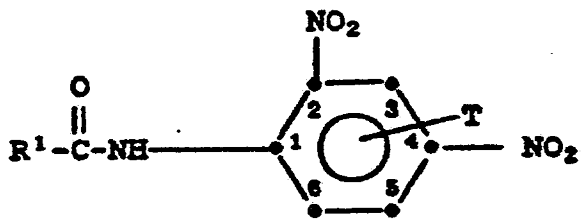

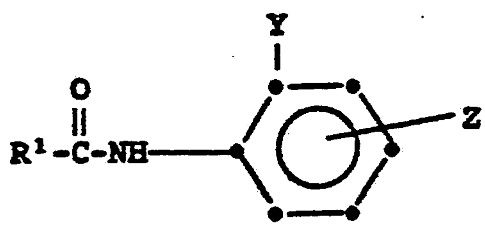

ou un sel de celui-ci;
dans laquelle $R^1$ représente

(1) un radical perfluorocyclopropyle
(2) un radical perfluorocyclobutyle
(3) un radical perfluorocycloheptyle
(4) un radical perfluorodécahydronaphtyle
(5) un radical fluorocyclopentyle suivant la formule

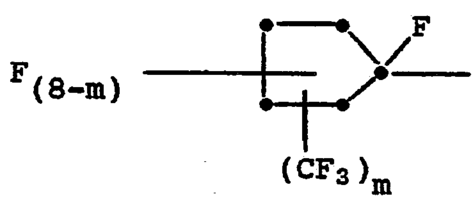

dans laquelle m représenté 0, 1 ou 2, ou
(6) un radical fluorocyclohexyle suivant la formule

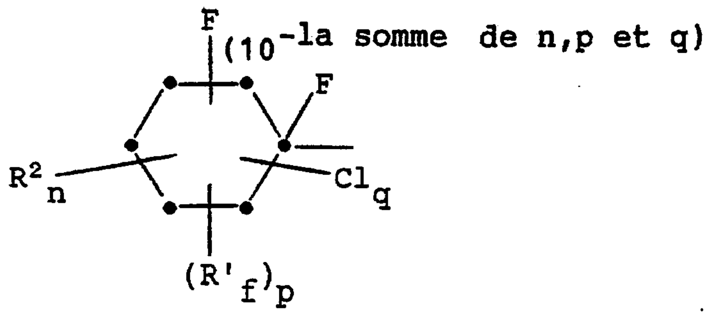

dans laquelle
$R^2$ représente $-OR_f$ ou

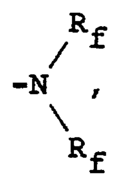

chaque $R_f$ représentant, indépendamment l'un de l'autre, un radical alkyle inférieur perfluoré en $C_1$–$C_3$, chaque $R'_f$ représentant, indépendamment l'un de l'autre, un radical alkyle inférieur perfluoré en $C_1$–$C_4$, n représentant 0 ou 1, chaque p et q représentant, indépendamment l'un de l'autre, 0, 1 ou 2, et la somme de n, p et q étant comprise entre 0 et 2, inclusivement;
$R^4$ est un radical thiocyanatophényle portant un seul substituant d'addition qui est un radical fluoro, chloro, bromo, iodo ou nitro; chaque X représente un halogène choisi indépendamment, excepté que pas plus d'un X ne peut représenter un radical iodo, T représente un radical alcoxy en $C_1$–$C_4$ ou un radical alkylthio en $C_1$–$C_4$ situé en position 3 ou 5, Y représente un halogène, un radical nitro ou méthyle, Z représente un radical nitro ou fluorosulfonyle et le sel est un sel sodique, potassique ou ammonique dans lequel l'ammonium présente la formule suivante

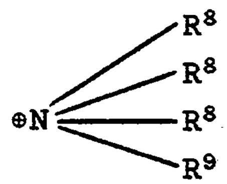

dans laquelle chaque $R^8$ représente indépendamment un radical alkyle en $C_1$–$C_{20}$, un radical benzyle, un radical 2-hydroxyéthyle, un radical 2-hydroxypropyle ou un radical 3-hydroxypropyle et $R^9$ représente de l'hydrogène ou $R^8$, le nombre total d'atomes de carbone dans tous les groupes $R^8$ et $R^9$ étant compris entre 12 et 60 sauf que si un ou plusieurs $R^8$ représentent un radical 2-hydroxyéthyle, un radical 2-hydroxypropyle ou un radical 3-hydroxypropyle, le nombre total d'atomes de carbone dans tous les groupes $R^8$ et $R^9$ est compris entre 6 et 60.

12. Procédé selon la revendication 11, dans lequel l'agent actif est le 2',4',6'-trichloro-1,2,2,3,3,4,4,5,5,6,6-undécafluorocyclohexanecarboxanilide ou un sel de celui-ci.

13. Procédé selon la revendication 11, dans lequel l'agent actif est le 2',4'-dinitro-5'-fluoro-1,2,2,3,3,4,4,5,5,6,6-undécafluorocyclohexanecarboxanilide ou un sel de celui-ci.

14. Procédé selon la revendication 11, dans lequel l'agent actif est le 2'-5'-dinitro-1,2,2,3,3,4,4,5,5,6,6-undécafluorocyclohexanecarboxanilide ou un sel de celui-ci.

15. Formulation insecticide ou arachnicide comprenant l'ingrédient actif décrit dans l'une quelconque des revendications 1 à 7, en combinaison avec un adjuvant acceptable en agriculture.

16. Formulation nématocide comprenant un agent actif tel que décrit à la revendication 10, en combinaison avec un adjuvant acceptable en agriculture.

17. Formulation herbicide comprenant un agent actif tel que décrit dans l'une des revendications 1 à 7, en combinaison avec un adjuvant acceptable en agriculture.

18. Formulation antifongique comprenant un agent actif tel que décrit dans l'une quelconque des revendications 1 à 14, en combinaison avec un adjuvant acceptable en agriculture.

19. Procédé pour préparer un composé de la formule (I) ou un sel de celui-ci tel que défini à la revendication 1, qui comprend

la réaction d'un halogénure d'acyle de la formule

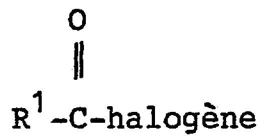

avec de l'aniline, du 1-aminonaphtalène ou de la 2-amino-nitropyridine suivant la formule

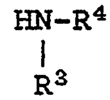

dans un solvant organique non réactif, et
si nécessaire, la méthylation du composé résultant de la formule (I) afin d'obtenir un composé de la formule (I) dans laquelle $R^3$ représente un radical méthyle, et
si nécessaire, la salification du composé résultant.